(19) Europäisches Patentamt / European Patent Office / Office européen des brevets

(11) **EP 3 967 322 B1**

(12) **EUROPEAN PATENT SPECIFICATION**

(45) Date of publication and mention
of the grant of the patent:
**05.03.2025 Bulletin 2025/10**

(21) Application number: **20805941.0**

(22) Date of filing: **11.05.2020**

(51) International Patent Classification (IPC):
*A61K 38/48* (2006.01)   *A61K 38/49* (2006.01)
*A61P 25/00* (2006.01)

(52) Cooperative Patent Classification (CPC):
**A61P 25/00; A61K 38/484; C12Y 304/21007**

(86) International application number:
**PCT/CN2020/089632**

(87) International publication number:
**WO 2020/228681 (19.11.2020 Gazette 2020/47)**

(54) **PLASMINOGEN FOR TREATING AMYOTROPHIC LATERAL SCLEROSIS**

PLASMINOGEN ZUR BEHANDLUNG VON AMYOTROPHER LATERALSKLEROSE

PLASMINOGÈNE POUR LE TRAITEMENT DE LA SCLÉROSE LATÉRALE AMYOTROPHIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **10.05.2019  PCT/CN2019/086431**

(43) Date of publication of application:
**16.03.2022  Bulletin 2022/11**

(73) Proprietor: **Talengen International Limited
Hong Kong 999077 (CN)**

(72) Inventor: **LI, Jinan
Shenzhen City, Guangdong 518020 (CN)**

(74) Representative: **karo IP
Patentanwälte PartG mbB
Steinstraße 16-18
40212 Düsseldorf (DE)**

(56) References cited:
**WO-A1-2009/146178     WO-A1-2009/146178
WO-A1-2010/124185     WO-A2-01/24784**

WO-A2-01/24784     WO-A2-2006/136419
WO-A2-2009/036336     WO-A2-2012/145428
CN-A- 102 482 338     US-A1- 2004 203 101
US-A1- 2011 086 894

• GLAS ET AL: "A role for the urokinase-type
plasminogen activator system in amyotrophic
lateral sclerosis", EXPERIMENTAL
NEUROLOGY, ELSEVIER, AMSTERDAM, NL, vol.
207, no. 2, 22 September 2007 (2007-09-22),
pages 350 - 356, XP022265533, ISSN: 0014-4886,
DOI: 10.1016/J.EXPNEUROL.2007.07.007
• M. FLINT BEAL: "The Urokinase System of
Plasminogen Activator Plays a Role in
Amyotrophic Lateral Sclerosis (ALS)
Pathogenesis", EXPERIMENTAL NEUROLOGY,
vol. 211, no. 2, 14 March 2008 (2008-03-14),
XP022668221, DOI: 20200810160920

Remarks:
The complete document including Reference
Table(s) and the Sequence Listing(s) can be
downloaded from the EPO website

**Description**

TECHNICAL FIELD

The present invention relates to plasminogen for use in a method for treating amyotrophic lateral sclerosis including administering an effective amount of plasminogen to a subject suffering from amyotrophic lateral sclerosis thereby repairing damaged nerves and improving clinical symptoms and signs. The invention is defined in the claims.

BACKGROUND ART

Amyotrophic lateral sclerosis (ALS), also known as gradual freezing disease, is a fatal neurodegenerative disease, mainly involving the pyramidal tract, brain stem, and anterior horn cells of spinal cord, and the clinical manifestations are progressively worsening muscle atrophy, acratia and spasm. More than 60% of patients died of respiratory muscle paralysis 3-5 years after the onset of this disease (Kiernan MC, Vucis S, Cheah BC, et al. Amyotrophic lateral sclerosis. Lancet, 2011, 377:942-955).

The clinical manifestations of amyotrophic lateral sclerosis are upper motor neuron degeneration (mainly characterized by tendon hyperreflexia and increased muscle tone) and lower motor neuron degeneration (muscular atrophy, muscle weakness, fasciculation and loss of tendon reflexes) as the main symptoms and signs. Generally, symptoms are asymmetrical, gradually progressing from the lesion location to other sites, but the extraocular muscles and sphincter muscles are mostly not involved. Although some patients may have mild sensory symptoms, usually the sensory system is tested as negative. Traditionally, it is believed that the cognitive function of patients with amyotrophic lateral sclerosis is well preserved. However, with the development of diagnostic techniques such as neuroimaging and neuropsychology, it has been found that impaired cognitive function is also a common feature of amyotrophic lateral sclerosis.

The prevalence of this disease is about 4-6/100,000. At present, the only therapeutic drug is the excitatory amino acid antagonist Rilutek, which has been approved by the drug regulatory authorities of various countries, but it can only slow the progression of the disease.

SUMMARY OF THE INVENTION

The research of the present invention finds that, plasminogen pathway activators such as plasminogen can significantly ameliorate motor neuron damage in the anterior horn of spinal cord, treat ALS, and ameliorate the symptoms of ALS.

The present invention relates to the following items:

A pharmaceutical composition comprising a therapeutically effective amount of a plasminogen pathway activator for use in treating amyotrophic lateral sclerosis (ALS), in a subject suffering from amyotrophic lateral sclerosis (ALS), wherein the plasminogen pathway activator is plasminogen.

In another aspect, which is not encompassed by the wording of the claims, the invention relates to: A method for treating amyotrophic lateral sclerosis (ALS), comprising administering to a subject suffering from amyotrophic lateral sclerosis (ALS) a therapeutically effective amount of one or more plasminogen pathway activators selected from the group consisting of: a component of the plasminogen activation pathway, a compound that can directly activate plasminogen or indirectly activate plasminogen by activating a upstream component of the plasminogen activation pathway, a compound that mimics plasminogen or its activity, a compound capable of up-regulating the expression of plasminogen or the plasminogen activator, a plasminogen analog, a plasmin analogs, a tPA or uPA analog, and an antagonist of fibrinolytic inhibitor.

In another aspect, which is not encompassed by the wording of the claims, the component of the plasminogen activation pathway is selected from the group consisting of: plasminogen, recombinant human plasmin, Lys-plasminogen, Glu-plasminogen, plasmin, plasminogen and plasmin variant and analog comprising one or more kringle domains and protease domains of plasminogen and plasmin, mini-plasminogen, mini-plasmin, micro-plasminogen, micro-plasmin, delta-plasminogen, delta-plasmin, plasminogen activator, tPA and uPA.

In another aspect, which is not encompassed by the wording of the claims, the antagonist of the fibrinolysis inhibitor is an antagonist of PAI-1, complement C1 inhibitor, $\alpha$2-antiplasmin or $\alpha$2-macroglobulin, e.g., an antibody to PAI-1, complement C1 inhibitor, $\alpha$2-antiplasmin, or $\alpha$2-macroglobulin.

In another aspect of the invention, the amyotrophic lateral sclerosis includes genetic and sporadic ALS.

In another aspect, the pharmaceutical composition of the present invention as described above, wherein the plasminogen pathway activator has one or more activities in the subject suffering from amyotrophic lateral sclerosis (ALS) and one or more activities are selected from the group consisting of: prolonging life span and median survival, delaying muscle atrophy and muscle strength loss, slowing down the rate of weight loss, reducing cell damage, degeneration and necrosis in the anterior horn of spinal cord, promoting the synthesis of chAT in the anterior horn of spinal cord, promoting the recovery of cholinergic neuron function, promoting the expression of synaptophysin in the anterior horn of spinal cord, promoting the expression of SMN protein in the anterior horn of spinal cord, promoting the repair of inflammation in the anterior horn of spinal cord, and promoting the repair of synaptic damage.

In another aspect, the pharmaceutical composition of the present invention as described above, wherein the plasminogen pathway activator ameliorates the symptoms of muscle atrophy, muscle strength loss, spasm, and/or fasciculation in the subject.

In another aspect, the pharmaceutical composition of the present invention as described above, wherein the plasminogen pathway activator reduces weight loss and/or prolongs survival in the subject.

In another aspect, the pharmaceutical composition of the present invention as described above, wherein the plasminogen pathway activator improves muscle tone in the subject.

In another aspect, the pharmaceutical composition of the present invention as described above, wherein the plasminogen pathway activator promotes the recovery of muscle function in the subject.

In another aspect, the pharmaceutical composition of the present invention as described above, wherein the plasminogen pathway activator promotes the repair of neuron damage in the anterior horn of spinal cord in the subject.

In another aspect, the pharmaceutical composition of the present invention as described above, wherein the plasminogen pathway activator is administered in combination with one or more other medicaments and/or therapies, preferably, the therapy includes cell therapy (e.g., stem cell therapy) and gene therapy, antisense RNA, small molecule splicing modifier, etc.

In another aspect, the pharmaceutical composition of the present invention as described above, wherein the plasminogen has at least 80%, 85%, 90%, 95%, 96%, 97%, 98% or 99% sequence identity with SEQ ID NOs: 2, 6, 8, 10 or 12, and has plasminogen activity.

In another aspect, the pharmaceutical composition of the present invention as described above, wherein the plasminogen is a protein that is an active fragment of plasminogen and has plasminogen activity and/or lysine binding activity.

In another aspect, the pharmaceutical composition of the present invention as described above, wherein the plasminogen is selected from the group consisting of: Glu-plasminogen, Lys-plasminogen, mini-plasminogen, micro-plasminogen, delta-plasminogen, and their variants retaining plasminogen activity.

In another aspect, the pharmaceutical composition of the present invention as described above, wherein the plasminogen is natural or synthetic human plasminogen, or a variant or fragment thereof retaining plasminogen activity and/or lysine binding activity.

In another aspect, the pharmaceutical composition of the present invention as described above, wherein the plasminogen is administered by intravenous, subcutaneous, intramuscular, intrathecal, nasal inhalation, aerosol inhalation, nasal drop or eye drop administration.

In any of the above-mentioned embodiments of the application, the plasminogen has at least 75%, 80%, 85%, 90%, 95%, 96%, 97%, 98% or 99% sequence identity with SEQ ID NOs: 2, 6, 8, 10 or 12, and still has plasminogen activity and/or lysine binding activity. In some embodiments, the plasminogen is a protein with 1-100, 1-90, 1-80, 1-70, 1-60, 1-50, 1-45, 1-40, 1-35, 1-30, 1-25, 1-20, 1-15, 1-10, 1-5, 1-4, 1-3, 1-2, or 1 amino acid addition, deletion and/or substitution on the basis of SEQ ID NOs: 2, 6, 8, 10 or 12, and still having plasminogen activity and/or lysine binding activity.

In some embodiments, the plasminogen is a protein comprising a fragment with plasminogen activity and/or lysine binding activity, and still has plasminogen activity and/or lysine binding activity. In some embodiments, the plasminogen is selected from the group consisting of: Glu-plasminogen, Lys-plasminogen, mini-plasminogen, micro-plasminogen, delta-plasminogen, or their variants retaining plasminogen activity and/or lysine binding activity. In some embodiments, the plasminogen is natural or synthetic human plasminogen, or a variant or fragment thereof that still retains plasminogen activity and/or lysine binding activity. In some embodiments, the plasminogen is a human plasminogen ortholog from a primate or rodent, or a variant or fragment thereof that still retains plasminogen activity and/or lysine binding activity. In some embodiments, the amino acid sequence of the plasminogen is shown in SEQ ID NOs: 2, 6, 8, 10 or 12. In some embodiments, the plasminogen is natural human plasminogen.

In some embodiments, the subject is human. In some embodiments, the subject is deficient in or lacks plasminogen. In some embodiments, the deficiency or lack is congenital, secondary and/or local.

In some embodiments of the foregoing methods, the plasminogen is administered systemically or locally. In some embodiments, the plasminogen is administered by nasal inhalation, aerosol inhalation, nasal drop or eye drop administration. In some embodiments, the plasminogen is administered by intravenous, intramuscular, subcutaneous, or intrathecal injection for treatment. In some embodiments of the foregoing method, the plasminogen is administered at a dose of 0.0001-2000 mg/kg, 0.001-800 mg/kg, 0.01-600 mg/kg, 0.1-400 mg/kg, 1-200 mg/kg, 1-100 mg/kg, 10-100 mg/kg (calculated by per kilogram of body weight); or 0.0001-2000 $mg/cm^2$, 0.001-800 $mg/cm^2$, 0.01-600 $mg/cm^2$, 0.1-400 $mg/cm^2$, 1-200$mg/cm^2$, 1-100 $mg/cm^2$, 10-100 $mg/cm^2$ (calculated by per square centimeter of body surface area), repeated one or more times, preferably administered at least every day, every two days, and every three days.

In some embodiments, which is not encompassed by the wording of the claims, the application relates to the following embodiments

A method for treating amyotrophic lateral sclerosis (ALS), comprising administering a therapeutically effective amount of a plasminogen pathway activator to a subject suffering from amyotrophic lateral sclerosis (ALS).

In another embodiment, which is not encompassed by the wording of the claims, the method according to the above-mentioned item, wherein the plasminogen pathway activator has one or more activities in the subject suffering from amyotrophic lateral sclerosis (ALS), and one or more activities are selected from the group consisting of prolonging life span and median survival, delaying muscle atrophy and muscle strength loss, slowing down the rate of weight loss, reducing cell damage, degeneration and necrosis in the anterior horn of spinal cord, promoting the synthesis of chAT in the anterior horn of spinal cord, promoting the recovery of cholinergic neuron function, promoting the expression of synaptophysin in the anterior horn of spinal cord, promoting the expression of SMN protein in the anterior horn of spinal cord, promoting the repair of inflammation in the anterior horn of spinal cord, and promoting the repair of synaptic damage.

In another embodiment, which is not encompassed by the wording of the claims, the method according to the above-mentioned item, wherein the plasminogen pathway activator ameliorates the symptoms of muscle atrophy, muscle strength loss, spasm, and/or fasciculation in the subject.

In another embodiment, which is not encompassed by the wording of the claims, the method according to the above-mentioned item wherein the plasminogen pathway activator reduces weight loss and/or prolongs survival in the subject.

In another embodiment, which is not encompassed by the wording of the claims, the method according to the above-mentioned item wherein the plasminogen pathway activator improves muscle tone in the subject.

In another embodiment, which is not encompassed by the wording of the claims, the method according to the above-mentioned item, wherein the plasminogen pathway activator promotes the recovery of muscle function in the subject.

In another embodiment, which is not encompassed by the wording of the claims, the method according to the above-mentioned item, wherein the plasminogen pathway activator promotes the repair of neuron damage in the anterior horn of spinal cord in the subject.

In another embodiment, which is not encompassed by the wording of the claims, the method according to the above-mentioned item, wherein the plasminogen pathway activator is administered in combination with one or more other medicaments and/or therapies.

In another embodiment, which is not encompassed by the wording of the claims, the method according to the above-mentioned item, wherein the plasminogen pathway activator is administered by intravenous, subcutaneous, intramuscular, intrathecal, nasal inhalation, aerosol inhalation, nasal drop or eye drop administration.

In another embodiment, which is not encompassed by the wording of the claims, the method according to the above-mentioned item, wherein the plasminogen pathway activator is a component of the plasminogen activation pathway.

In another embodiment, which is not encompassed by the wording of the claims, the method according to the above-mentioned item, wherein the component of the plasminogen activation pathway is plasminogen.

In another embodiment, which is not encompassed by the wording of the claims, the method according to the above-mentioned item, wherein the plasminogen has at least 80%, 85%, 90%, 95%, 96%, 97%, 98% or 99% sequence identity with SEQ ID NOs: 2, 6, 8, 10 or 12, and has plasminogen activity.

In another embodiment, which is not encompassed by the wording of the claims, the method according to the above-mentioned item, wherein the plasminogen is a protein that is an active fragment of plasminogen and has plasminogen activity and/or lysine binding activity.

In another embodiment, which is not encompassed by the wording of the claims, the method according to the above-mentioned item, wherein the plasminogen is selected from the group consisting of Glu-plasminogen, Lys-plasminogen, mini-plasminogen, micro-plasminogen, delta-plasminogen, and their variants retaining plasminogen activity.

In another embodiment, which is not encompassed by the wording of the claims, the method according to the above-mentioned item, wherein the plasminogen is natural or synthetic human plasminogen, or a variant or fragment thereof retaining plasminogen activity and/or lysine binding activity.

The present invention also relates to a pharmaceutical composition, medicament, formulation, kit, and product for treating amyotrophic lateral sclerosis (ALS), which comprises a therapeutically effective amount of the plasminogen pathway activator.

In some embodiments, the plasminogen pathway activator has one or more activities selected from the group consisting of: prolonging life span and median survival, delaying muscle atrophy and muscle strength loss, slowing down the rate of weight loss, reducing cell damage, degeneration and necrosis in the anterior horn of spinal cord, promoting the synthesis of chAT in the anterior horn of spinal cord, promoting the recovery of cholinergic neuron function, promoting the expression of synaptophysin in the anterior horn of spinal cord, promoting the expression of SMN protein in the anterior horn of spinal cord, promoting the repair of inflammation in the anterior horn of spinal cord, and promoting the repair of synaptic damage. In some embodiments, the plasminogen pathway activator ameliorates the symptoms of muscle atrophy, muscle strength loss, spasm, and/or fasciculation in the subject. In some embodiments, the plasminogen pathway activator reduces weight loss and/or prolongs survival in the subject. In some embodiments, the plasminogen pathway activator improves muscle tone in the subject. In some embodiments, the plasminogen pathway activator promotes the recovery of muscle function in the subject. In some embodiments, the plasminogen pathway activator promotes the repair of neuron damage in the anterior horn of spinal cord in the subject.

In some embodiments, the plasminogen pathway activator is a component of the plasminogen activation pathway. In some embodiments, the component of the plasminogen activation pathway is plasminogen. In some embodiments, the plasminogen has at least 80%, 85%, 90%, 95%, 96%, 97%, 98% or 99% sequence identity with SEQ ID NOs: 2, 6, 8, 10 or 12, and still has plasminogen activity and/or lysine binding activity. In some embodiments, the plasminogen is a protein comprising a fragment with plasminogen activity and/or lysine binding activity and still having plasminogen activity and/or lysine binding activity. In some embodiments, the plasminogen is selected from the group consisting of: Glu-plasminogen, Lys-plasminogen, mini-plasminogen, micro-plasminogen, delta-plasminogen, or their variants retaining plasminogen activity. In some embodiments, the plasminogen is natural or synthetic human plasminogen, or a variant or fragment thereof that still retains plasminogen activity and/or lysine binding activity.

In some embodiments, the plasminogen pathway activator, for example, a component of the plasminogen activation pathway, such as plasminogen, is administered in combination with one or more other medicaments and/or therapies. In some embodiments, the plasminogen pathway activator, for example, a component of the plasminogen activation pathway, such as plasminogen, is administered by intravenous, intramuscular, subcutaneous, intrathecal, nasal inhalation, aerosol inhalation, nasal drop or eye drop administration.

In some embodiments, the pharmaceutical composition, medicament, or formulation comprises a pharmaceutically acceptable carrier and a plasminogen pathway activator, for example, a component of the plasminogen activation pathway, such as plasminogen. In some embodiments, the kit and product comprise one or more containers comprising the pharmaceutical composition, medicament, or formulation. In some embodiments, the kit or product further comprises a label or instructions for use, and the label or instructions for use indicate a method for treating amyotrophic lateral sclerosis by using a plasminogen pathway activator, for example, a component of the plasminogen activation pathway, such as plasminogen.

In some embodiments, the kit or product further comprises one or more additional containers comprising other medicaments.

The present invention also relates to use of a therapeutically effective amount of plasminogen pathway activator in the preparation of a pharmaceutical composition, medicament, formulation, kit, and product for treating amyotrophic lateral sclerosis (ALS).

In some embodiments, the plasminogen pathway activator has one or more activities in the subject suffering from amyotrophic lateral sclerosis (ALS), and one or more activities are selected from the group consisting of: prolonging life span and median survival, delaying muscle atrophy and muscle strength loss, slowing down the rate of weight loss, reducing cell damage, degeneration and necrosis in the anterior horn of spinal cord, promoting the synthesis of chAT in the anterior horn of spinal cord, promoting the recovery of cholinergic neuron function, promoting the expression of synaptophysin in the anterior horn of spinal cord, promoting the expression of SMN protein in the anterior horn of spinal cord, promoting the repair of inflammation in the anterior horn of spinal cord, and promoting the repair of synaptic damage. In some embodiments, the plasminogen pathway activator ameliorates the symptoms of muscle atrophy, muscle strength loss, spasm, and/or fasciculation in the subject. In some embodiments, the plasminogen pathway activator reduces weight loss and/or prolongs survival in the subject. In some embodiments, the plasminogen pathway activator improves muscle tone in the subject. In some embodiments, the plasminogen pathway activator promotes the recovery of muscle function in the subject. In some embodiments, the plasminogen pathway activator promotes the repair of neuron damage in the anterior horn of spinal cord in the subject.

In some embodiments, the plasminogen pathway activator is a component of the plasminogen activation pathway. In some embodiments, the component of the plasminogen activation pathway is plasminogen. In some embodiments, the plasminogen has at least 75%, 80%, 85%, 90%, 95%, 96%, 97%, 98% or 99% sequence identity with SEQ ID NOs: 2, 6, 8, 10 or 12, and still has plasminogen activity and/or lysine binding activity. In some embodiments, the plasminogen is a protein comprising a fragment with plasminogen activity and/or lysine binding activity and still having plasminogen activity and/or lysine binding activity. In some embodiments, the plasminogen is selected from the group consisting of: Glu-plasminogen, Lys-plasminogen, mini-plasminogen, micro-plasminogen, delta-plasminogen, or their variants retaining plasminogen activity. In some embodiments, the plasminogen is natural or synthetic human plasminogen, or a variant or fragment thereof that still retains plasminogen activity and/or lysine binding activity.

In some embodiments, the plasminogen pathway activator, for example, a component of the plasminogen activation pathway, such as plasminogen, is administered in combination with one or more other medicaments and/or therapies. In some embodiments, the plasminogen pathway activator, for example, a component of the plasminogen activation pathway, such as plasminogen, is administered by intravenous, intramuscular, subcutaneous, intrathecal, nasal inhalation, aerosol inhalation, nasal drop or eye drop administration.

In some embodiments, the pharmaceutical composition, medicament, or formulation comprises a pharmaceutically acceptable carrier and a plasminogen pathway activator, for example, a component of the plasminogen activation pathway, such as plasminogen. In some embodiments, the kit and product comprise one or more containers comprising the pharmaceutical composition, medicament, or formulation. In some embodiments, the kit or product further comprises a label or instructions for use, and the label or instructions for use indicate a method for treating amyotrophic lateral sclerosis by using plasminogen pathway activator, for example, a component of the plasminogen activation pathway, such as plasminogen.

In some embodiments, the kit or product further comprises one or more additional containers comprising other medicaments.

## BRIEF DESCRIPTION OF THE DRAWINGS

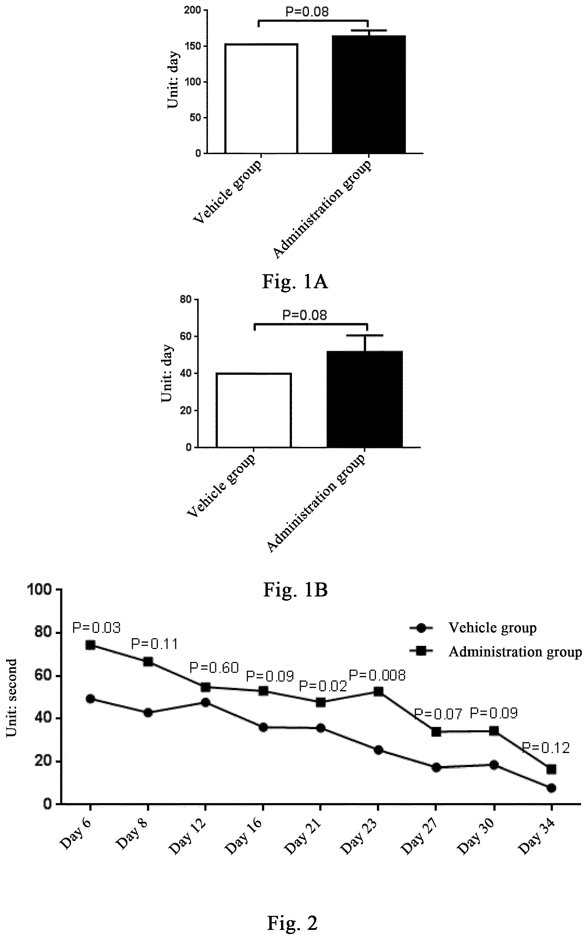

Fig. 1 shows the statistical results of the lifespan and survival time of ALS model mice after administration of plasminogen. Fig. 1A shows the statistical results of life span, and Fig. 1B shows the statistical results of survival time. The results show that, the average life span of the mice in the plasminogen group is 164 ±8.6 days, the average life span of the mice in the vehicle control group is 153±0 days, and the life span of the mice in the plasminogen group is about 11 days longer than that of the mice in the vehicle control group; the median survival time of the mice in the plasminogen group is 53±9 days, the median survival time of the mice in the vehicle control group is 40±0 days, and the median survival time of the mice in the plasminogen group is about 13 days longer than that of the mice in the vehicle control group, about 30% longer. This result indicates that, plasminogen can prolong the lifespan and median survival of ALS mice.

The results in Fig. 2 show that, although the suspension latent time of the mice in the two groups is decreasing during the administration period, the suspension latent time of the mice in the plasminogen group is always longer than that of the mice in the vehicle control group, and on the day 6, 21, and 23 of administration, comparing the suspension latent time of the plasminogen group with that of the vehicle group, the statistical difference is significant or extremely significant, with the P values of 0.03, 0.02, and 0.008, respectively. It indicates that plasminogen can delay the muscle strength loss of ALS mice.

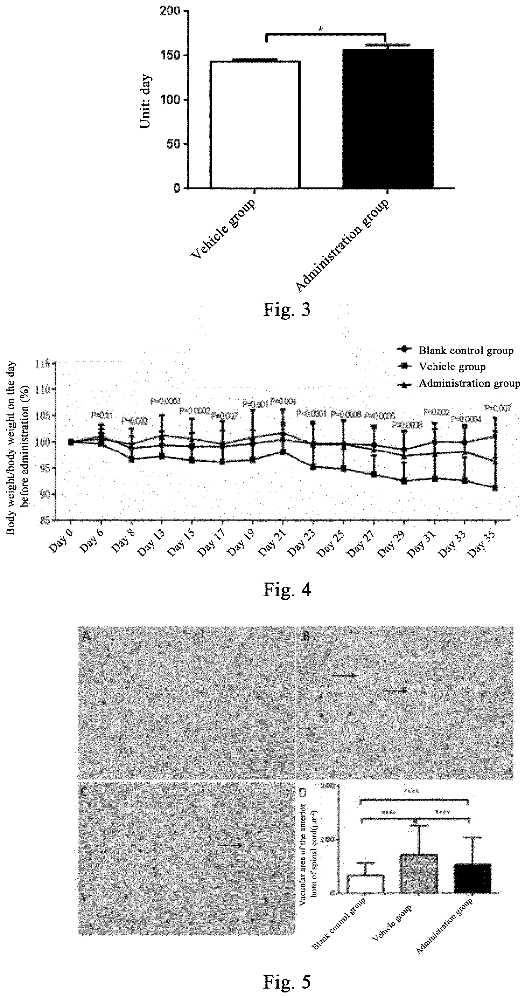

Fig. 3 shows the time of neurobehavioral manifestation with a score of 2 in ALS model mice after administration of plasminogen. The results show that, the time point of exhibiting neurobehavioral manifestation with a score of 2 for the mice in the plasminogen group is significantly later than that for the mice in the vehicle group, and the statistical difference is significant (* means P<0.05).

Fig. 4 shows the statistical results of the percentage of body weight of normal mice and ALS model mice relative to their body weight on day 1, after plasminogen administration. The results show that during administration, the weight of the mice in the blank control group does not fluctuate much with a gradual upward trend; the weight of the mice in the vehicle control group gradually decreases; the weight of the mice in the plasminogen group fluctuates greatly in the first 25 days, but all is close to or slightly larger than the body weight of the mice in the blank control group, then the body weight gradually decreases after 25 days, but it is always greater than that of the mice in the vehicle control group, and compared with the vehicle control group, the P value is less than or close to 0.001. It indicates that, plasminogen can significantly reduce the rate of weight loss in ALS model mice, and delay the deterioration of ALS.

Fig. 5 shows the statistical results of the vacuolar area in the H&E staining of the anterior horn of spinal cord of normal mice and ALS model mice, after plasminogen administration. A is the blank control group, B is the vehicle group, C is the administration group, and D is the statistical result of the vacuolar area. The results show that, the anterior horn of spinal cord of the blank control mice exhibits a certain level of vacuolar area, and the vacuolar area of the anterior horn of spinal cord of the mice in the vehicle group is significantly larger than that of the mice in the blank control (P<0.001); the vacuolar area of the anterior horn of spinal cord of the mice in the administration group is significantly lower than that of the mice in the vehicle group, and the statistical difference is extremely significant. It is suggested that, plasminogen can reduce the vacuolar area of the anterior horn of spinal cord, and reduce the death of motor neurons in the anterior horn of spinal cord in ALS model mice.

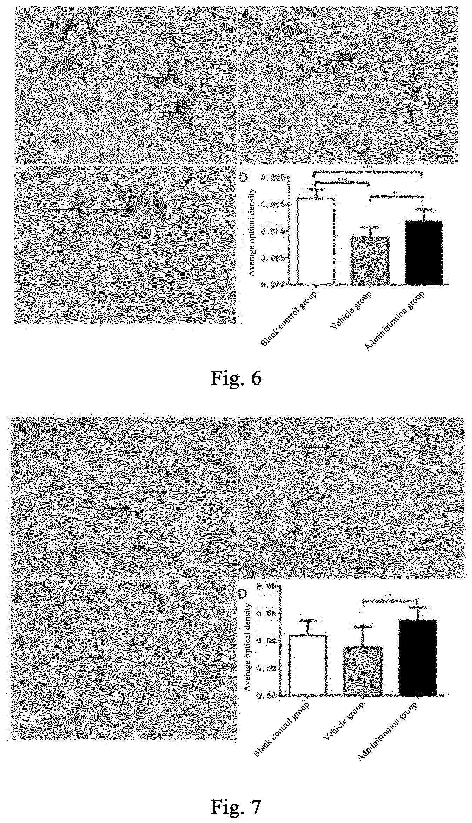

Fig. 6 shows the immunohistochemical staining results of chAT in the anterior horn of spinal cord of normal mice and ALS model mice, after administration of plasminogen. A is the blank control group, B is the vehicle group, C is the administration group, and D is the statistical result of the average optical density. The results show that, a certain amount of chAT is expressed in the anterior horn of spinal cord in the blank control mice, the expression level of chAT in the anterior horn of spinal cord of the mice in the vehicle group is significantly lower than that of the mice in the blank control group, the expression level of chAT in the anterior horn of spinal cord of the mice in the administration group is significantly higher than that of the mice in the vehicle group, and the statistical difference is significant (P<0.05). It indicates that plasminogen can promote the synthesis and expression of chAT in the anterior horn of spinal cord of SOD1-G93A mice, and promote the recovery of cholinergic neuron function.

Fig. 7 shows the results of immunohistochemical staining of synaptophysin in the anterior horn of spinal cord of normal mice and ALS model mice, after administration of plasminogen. A is the blank control group, B is the vehicle group, C is the administration group, and D is the statistical result of the average optical density. The results show that, a certain level of synaptophysin is expressed in the anterior horn of spinal cord of the mice in the blank control group, and the expression level of synaptophysin of the mice in the vehicle group is significantly lower than that of the mice in the blank control group; the expression level of synaptophysin in the anterior horn of spinal cord of the mice in the administration group is significantly higher than that of the mice in the vehicle group, and the statistical difference is significant (P<0.05). It indicates that plasminogen can promote the expression of synaptophysin in the anterior horn of spinal cord of model mice, and promote the repair of synaptic damage.

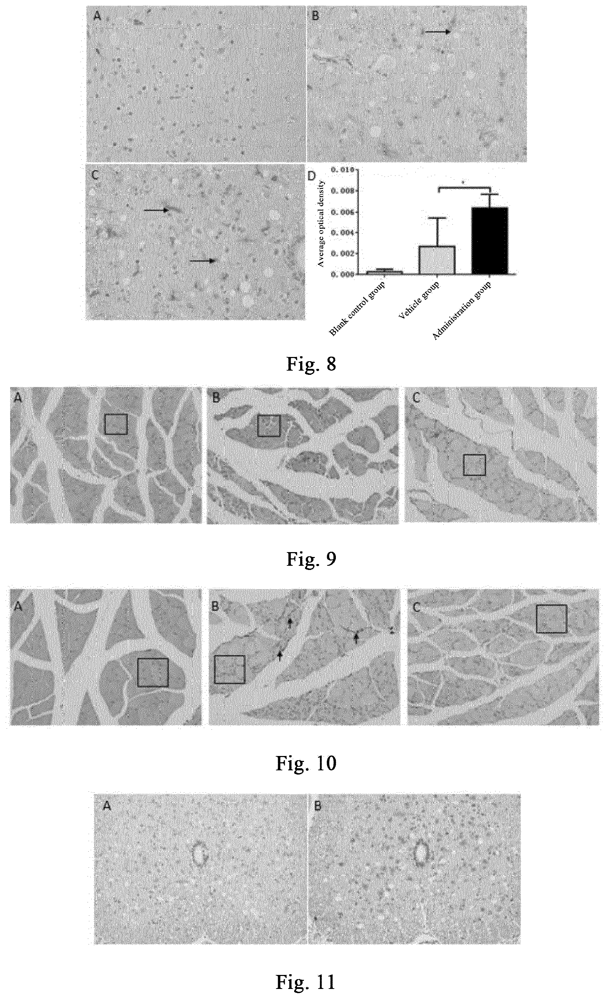

Fig. 8 shows the results of immunohistochemical staining of Iba-1 in the anterior horn of spinal cord of normal mice and ALS model mice, after administration of plasminogen. A is the blank control group, B is the vehicle group, C is the administration group, and D is the statistical result of the average optical density. The results show that, a certain level of Iba-1 is expressed in the anterior horn of spinal cord of the blank control mice, the expression level of Iba-1 in the anterior horn of spinal cord of the mice in the administration group is significantly higher than that of the mice in the vehicle group and the blank control group, and the statistical difference is significant (P<0.05 or 0.01). It indicates that plasminogen can promote the repair of inflammation in the anterior horn of spinal cord in the model mice.

Fig. 9 shows the representative pictures of H&E staining of gastrocnemius in normal mice and ALS model mice, after plasminogen administration. A is the blank control group, B is the vehicle group, and C is the administration group. The results show that, the gastrocnemius fibers in the blank control group are complete in structure and relatively uniform in shape and size, while the gastrocnemius fibers in the vehicle group show severe atrophy with local inflammatory cell infiltration (red arrow) and roundness change of muscle fibers; the muscle fiber atrophy in the administration group is less severe than that in the vehicle group, but there was also inflammatory cell infiltration. It indicates that, plasminogen can ameliorate muscle atrophy in the model mice.

Fig. 10 shows the representative image of H&E staining of the gluteal muscle of normal mice and ALS model mice, after plasminogen administration. A is the blank control group, B is the vehicle group, and C is the administration group. The results show that, the muscle fibers in the blank control group mice are relatively complete in structure, and relatively uniform in shape and size. The muscle fibers of gluteal muscle of the mice in the vehicle group show roundness change, different sizes, severe atrophy, and infiltration of inflammatory cells; the structure of the gluteal muscle fibers of the mice in the administration group recovers to a certain extent as compared with that in the vehicle group. It indicates that, plasminogen can ameliorate muscle atrophy in the model mice.

Fig. 11 shows the representative image of immunohistochemical staining of SMN protein in the anterior horn of spinal cord of ALS model mice, after plasminogen administration. A is the vehicle group, and B is the administration group. The results show that, the expression level of SMN protein in the anterior horn of spinal cord of the mice in the administration group is significantly higher than that of the mice in the vehicle group. It indicates that plasminogen can promote the expression of SMN protein in the anterior horn of spinal cord of model mice.

## DETAIL DESCRIPTION OF THE INVENTION

In the present invention, "amyotrophic lateral sclerosis" refers to a series of pathological changes caused by motor neuron damage. The pathological changes include motor neuron degeneration, gliosis, nerve fiber abnormality, loss of myelinated fibers in the corticospinal tract and anterior root of spinal nerve. The manifestations of medullary motor neuron damage, for example, include facial muscle, language and swallowing dysfunction; the manifestations of spinal cord motor neuron damage include muscle spasm, muscle weakness, muscle atrophy, paralysis, and respiratory failure.

ALS is characterized by progressive manifestations of dysfunction of the lower and upper motor neurons. Lower motor neurons connect the brainstem and spinal cord to muscle fibers, and their dysfunction leads to muscle atrophy, spasm, and fasciculation. Upper motor neurons originate from the motor area of the cerebral cortex or brainstem, and carry motor information to the motor neurons that directly respond to stimulate the target muscle. Their dysfunction leads to spasm (continuous muscle contractions that interfere with gait, movement, and speech) and pathological reflexes. ALS can be divided into sporadic ALS (sALS) and familial ALS (fALS) according to whether it has familial inheritance. There is no ALS family history for sporadic ALS patients, and there is more than one ALS patient in the family with familial ALS. According to the different ways of inheritance, familial ALS can be divided into autosomal dominant inheritance, autosomal recessive inheritance, and X chromosome linked inheritance.

Motor nerves can nourish muscle tissues. After cutting off the motor nerve, in the muscle the glycogen synthesis slows down, the protein decomposition accelerates, and the muscle gradually shrinks. The application also relates to the treatment of muscle atrophy caused by motor nerve damage and related disorders by using plasminogen.

Fibrinolytic system is a system composed of a series of chemical substances involved in the process of fibrinolysis, and the chemical substances mainly comprises plasminogen, plasmin, plasminogen activator, and fibrinolysis inhibitor. Plasminogen activator includes tissue-type plasminogen activator (t-PA), and urokinase-type plasminogen activator (u-PA). t-PA is a serine protease, which is synthesized by vascular endothelial cells. t-PA activates plasminogen, and this process is mainly carried out on fibrin; urokinase-type plasminogen activator (u-PA) is produced by renal tubular epithelial cells and vascular endothelial cells, and it can directly activate plasminogen, and does not require fibrin as a cofactor. Plasminogen (PLG) is synthesized by the liver. When the blood coagulates, PLG is adsorbed on the fibrin net in a large amount, and under the action of t-PA or u-PA it is activated into plasmin to promote fibrinolysis. Plasminase (PL) is a serine protease, which has the functions of: degrading fibrin and fibrinogen; hydrolyzing a variety of coagulation factors V, VIII, X, VII, XI, II, etc.; transforming plasminogen into plasmin; hydrolyzing a complement, etc. Fibrinolytic inhibitor includes: plasminogen activator inhibitor (PAI), and $\alpha$2-antiplasmin ($\alpha$2-AP). PAI mainly has two forms, PAI-1 and PAI-2, which can specifically bind to t-PA in a ratio of 1:1, thereby inactivating it and activating PLG at the same time. $\alpha$2-AP is synthesized by the liver, and bond with PL in a ratio of 1:1 to form a complex, thereby inhibiting PL activity; FXIII makes $\alpha$2-AP covalently bond with fibrin, thereby weakening the sensitivity of fibrin to PL. Substances inhibiting the activity of the fibrinolytic system *in vivo* comprise: PAI-1, complement C1 inhibitor; $\alpha$2 anti-plasmin; and $\alpha$2-macroglobulin.

The term "plasminogen pathway activator" or "profibrinolysin pathway activator" as used herein encompasses a component of the plasminogen activation pathway, a compound that can directly activate plasminogen or indirectly activate plasminogen by activating a upstream component of the plasminogen activation pathway, a compound that mimics plasminogen or its activity, a compound capable of up-regulating the expression of plasminogen or the plasminogen activator, a plasminogen analog, a plasmin analog, a tPA or uPA analog, and an antagonist of fibrinolytic inhibitor.

The term "component of the plasminogen activation pathway" or "component of the profibrinolysin activation pathway" as used herein encompasses:

1. plasminogen, Lys-plasminogen, Glu-plasminogen, micro-plasminogen, delta-plasminogen; and their variants or analogs;
2. plasmin and their variants or analogs; and
3. plasminogen activators, for example, tPA and uPA, and tPA or uPA variants and analogs comprising one or more domains of tPA or uPA (such as one or more kringle domains and proteolytic domains).

The term "antagonist of fibrinolysis inhibitor" encompasses antagonist of PAI-1, complement C1 inhibitor, $\alpha$2-antiplasmin or $\alpha$2-macroglobulin, for example, antibody of PAI-1, complement C1 inhibitor, $\alpha$2-antiplasmin or $\alpha$2-macroglobulin.

The above-mentioned "variants" of plasminogen, plasmin, tPA and uPA include all naturally occurring human genetic variants and other mammalian forms of these proteins, as well as a protein with, for example, 1-100, 1-90, 1-80, 1-70, 1-60, 1-50, 1-45, 1-40, 1-35, 1-30, 1-25, 1-20, 1- 15, 1-10, 1-5, 1-4, 1-3, 1-2, or 1 amino acid addition, deletion and/or substitution, and still having plasminogen, plasmin, tPA or uPA activity. For example, "variants" of plasminogen, plasmin, tPA, and uPA include mutant variants of these proteins with, for example, 1-100, 1-90, 1-80, 1-70, 1-60, 1-50, 1-45, 1-40, 1-35, 1-30, 1-25, 1-20, 1-15, 1-10, 1-5, 1-4, 1-3, 1-2, or 1 conservative amino acid substitution.

The term "plasminogen variant" as used herein encompasses a protein that has at least 75%, 80%, 85%, 90%, 95%, 96%, 97%, 98% or 99% sequence identity with SEQ ID NOs: 2, 6, 8, 10 or 12, and still has plasminogen activity. For example, the "plasminogen variant" of the present invention can be a protein with 1-100, 1-90, 1-80, 1-70, 1-60, 1-50, 1-45, 1-40, 1-35, 1-30, 1-25, 1-20, 1- 15, 1-10, 1-5, 1-4, 1-3, 1-2, or 1 amino acid addition, deletion and/or substitution on the basis of SEQ ID NOs: 2, 6, 8, 10 or 12, and still having plasminogen activity and/or lysine binding activity. Particularly, the plasminogen variants of the present invention include all naturally occurring human genetic variants and other mammalian forms of these proteins, as well as mutant variants of these proteins obtained by, for example, 1-100, 1-90, 1-80, 1-70, 1-60, 1-50, 1-45, 1-40, 1-35, 1-30, 1-25, 1-20, 1- 15, 1-10, 1-5, 1-4, 1-3, 1-2, or 1 conservative amino acid substitution.

The plasminogen of the present invention may be a human plasminogen ortholog from a primate or rodent, or a variant thereof still retaining plasminogen activity and/or lysine binding activity, for example, the plasminogen shown in SEQ ID NOs: 2, 6, 8, 10 or 12, such as the human natural plasminogen shown in SEQ ID NO: 2.

The above-mentioned "analogs" of plasminogen, plasmin, tPA and uPA, include compounds that provide substantially similar effects to plasminogen, plasmin, tPA, or uPA, respectively.

The above-mentioned "variants" and "analogs" of plasminogen, plasmin, tPA and uPA, encompass "variants" and "analogs" of plasminogen, plasmin, tPA and uPA comprising one or more domains (e.g., one or more kringle domains and proteolytic domains). For example, "variants" and "analogs" of plasminogen encompass variants and analogs of plasminogen comprising one or more plasminogen domains (e.g., one or more kringle domains and proteolytic domains), such as mini-plasminogen. "Variants" and "analogs" of plasmin encompass "variants" and "analogs" of plasmin comprising one or more plasmin domains (e.g., one or more kringle domains and proteolytic domains), such as mini-plasmin, and delta-plasmin.

Whether the above-mentioned "variants" or "analogs" of plasminogen, plasmin, tPA or uPA have the activity of plasminogen, plasmin, tPA or uPA respectively, or whether they provide substantially similar effects to plasminogen, plasmin, tPA or uPA can be detected by methods known in the art. For example, it can be measured by activated plasmin activity level based on enzymography, ELISA (enzyme-linked immunosorbent assay) and FACS (fluorescence-activated cell sorting method), for example, it can be measured with reference to a method selected from the following documents: Ny, A., Leonardsson, G. , Hagglund, A.C, Hagglof, P. , Ploplis, V.A., Carmeliet, P. and Ny, T. (1999). Ovulation inplasminogen-deficient mice. Endocrinology 140, 5030-5035; Silverstein RL, Leung LL, Harpel PC, Nachman RL (November 1984). "Complex formation of platelet thrombospondin with plasminogen. Modulation of activation by tissue activator". J. Clin. Invest. 74 (5): 1625-33; Gravanis I, Tsirka SE (February 2008). "Tissue-type plasminogen activator as a therapeutic target in stroke". Expert Opinion on Therapeutic Targets. 12 (2): 159-70; Geiger M, Huber K, Wojta J, Stingl L, Espana F, Griffin JH, Binder BR (Aug 1989). "Complex formation between urokinase and plasma protein C inhibitor in vitro and in vivo". Blood. 74 (2): 722-8.

In some embodiments of the present invention, the "component of plasminogen activation pathway" of the present invention is a plasminogen selected from the group consisting of: Glu-plasminogen, Lys-plasminogen, mini-plasminogen, micro-plasminogen, delta-plasminogen, or their variants retaining plasminogen activity. In some embodiments, the plasminogen is natural or synthetic human plasminogen, or a conservative mutant variant or fragment thereof that still retains plasminogen activity and/or lysine binding activity. In some embodiments, the plasminogen is a human plasminogen ortholog from a primate or rodent, or a conservative mutant variant or fragment thereof that still retains plasminogen activity and/or lysine binding activity. In some embodiments, the amino acid sequence of the plasminogen is shown in SEQ ID NOs: 2, 6, 8, 10 or 12. In some embodiments, the plasminogen is natural human plasminogen. In some embodiments, the plasminogen is human natural plasminogen as shown in SEQ ID NO: 2.

"Compound that can directly activate plasminogen or indirectly activate plasminogen by activating a upstream component of the plasminogen activation pathway" refers to any compound that can directly activate plasminogen or indirectly activate plasminogen by activating a upstream component of the plasminogen activation pathway, e.g., tPA, uPA, streptokinase, saruplase, alteplase, reteplase, tenecteplase, anistreplase, monteplase, lanoteplase, pamiteplase, and staphylokinase.

The "antagonist of the fibrinolysis inhibitor" of the present invention is a compound that antagonizes, weakens, blocks, and prevents the action of the fibrinolysis inhibitor. The fibrinolysis inhibitors are, for example, PAI-1, complement C1 inhibitor, α2-antiplasmin, and α2-macroglobulin. The antagonist is an antibody to PAI-1, complement C1 inhibitor, α2-antiplasmin or α2-macroglobulin; or antisense RNA or mini-RNA that blocks or down-regulates the expression of for example PAI-1, complement C1 inhibitor, α2-antiplasmin or α2-macroglobulin; or a compound that occupies the binding site of PAI-1, complement C1 inhibitor, α2-antiplasmin or α2-macroglobulin but without the function of PAI-1, complement C1 inhibitor, α2-antiplasmin or α2-macroglobulin; or a compound that blocks the binding domain and/or active domain of PAI-1, complement C1 inhibitor, α2-antiplasmin or α2-macroglobulin.

Plasmin is a key component of the plasminogen activation system (PA system). It is a broad-spectrum protease that can hydrolyze several components of the extracellular matrix (ECM), including fibrin, gelatin, fibronectin, laminin and proteoglycan. In addition, plasmin can activate some precursors of metalloproteinases (pro-MMPs) to form active metalloproteases (MMPs). Therefore, plasmin is considered to be an important upstream regulator of extracellular proteolysis. Plasmin is formed by proteolysis of plasminogen through two physiological PAs: tissue-type plasminogen activator (tPA) or urokinase-type plasminogen activator (uPA). Due to the relatively high levels of plasminogen in plasma and other body fluids, it is traditionally believed that the regulation of the PA system is mainly achieved through regulating the synthesis and activity levels of PAs. The synthesis of PA system components is strictly regulated by different factors, such as hormones, growth factors and cytokines. In addition, there are specific physiological inhibitors of plasmin and PAs. The main inhibitor of plasmin is α2-antiplasmin. The activity of PAs is inhibited by both uPA and tPA plasminogen activator inhibitor-1 (PAI-1), and regulated by plasminogen activator inhibitor-2 (PAI-2) that mainly inhibits uPA. Certain cell surfaces have uPA-specific cell surface receptors (uPAR) with direct hydrolytic activity.

Plasminogen is a single-chain glycoprotein consisting of 791 amino acids with a molecular weight of approximately 92 kDa. Plasminogen is mainly synthesized in the liver, and exists in large amounts in the extracellular fluid. The plasminogen content in plasma is about 2 μM. Therefore, plasminogen is a huge potential source of proteolytic activity in tissues and body fluids. Plasminogen exists in two molecular forms: Glu-plasminogen and Lys-plasminogen. The naturally secreted and uncleaved form of plasminogen has an amino terminal (N-terminal) glutamate, and is therefore called glutamate-plasminogen. However, in the presence of plasmin, glutamate-plasminogen is hydrolyzed at Lys76-Lys77 into lysine-plasminogen. Compared with glutamate-plasminogen, lysine-plasminogen has a higher affinity for fibrin, and can be activated by PAs at a higher rate. The Arg560-Val561 peptide bond of these two forms of plasminogen can be cleaved by uPA or tPA, resulting in the formation of a disulfide bond-linked double-chain protease plasmin. The amino terminal moiety of plasminogen comprises five homologous tricyclic rings, i.e., so-called kringles, and the carboxy terminal moiety comprises the protease domain. Some kringles comprise lysine binding sites that mediate the specific interaction of plasminogen with fibrin and its inhibitor α2-AP. A newly discovered plasminogen is a 38 kDa fragment, including kringles1-4, which is an effective inhibitor of angiogenesis. This fragment is named as angiostatin, and can be produced by the hydrolysis of plasminogen by several proteases.

The main substrate of plasmin is fibrin, and the dissolution of fibrin is the key to preventing pathological thrombosis. Plasmin also has substrate specificity for several components of ECM, including laminin, fibronectin, proteoglycan and gelatin, indicating that plasmin also plays an important role in ECM reconstruction. Indirectly, plasmin can also degrade other components of ECM by converting certain protease precursors into active proteases, including MMP-1, MMP-2, MMP-3 and MMP-9. Therefore, it has been suggested that plasmin may be an important upstream regulator of extracellular proteolysis. In addition, plasmin has the ability to activate certain latent forms of growth factors. Plasmin can also hydrolyze components of the complement system in vitro, and release chemotactic complement fragments.

"Plasmin" is a very important enzyme present in the blood, and it can hydrolyze fibrin clots into fibrin degradation products and D-dimers.

"Plasminogen" is the zymogen form of plasmin, referring to the sequence in swiss prot, it is a glycoprotein composed of 810 amino acids with a molecular weight of about 90 kD according to the amino acid sequence (SEQ ID NO: 4) of natural human plasminogen comprising a signal peptide, and this glycoprotein is mainly synthesized in the liver and able to circulate in the blood, and the cDNA sequence encoding this amino acid sequence is shown in SEQ ID NO: 3. The full-length plasminogen comprises seven domains: a serine protease domain at the C terminal, a Pan Apple (PAp) domain at the N terminal, and five Kringle domains (Kringle1-5). Referring to the sequence in swiss prot, its signal peptide comprises residues Met1-Gly19, PAp comprises residues Glu20-Val98, Kringle1 comprises residues Cys103-Cys181, Kringle2 comprises residues Glu184-Cys262, Kringle3 comprises residues Cys275-Cys352, Kringle4 comprises residues Cys377-Cys454, and Kringle5 comprises residues Cys481-Cys560. According to NCBI data, the serine protease domain comprises residues Val581-Arg804.

Glu-plasminogen is a natural full-length plasminogen consisting of 791 amino acids (without the signal peptide of 19 amino acids). The cDNA sequence encoding this sequence is shown in SEQ ID NO: 1, and its amino acid sequence is shown in SEQ ID NO: 2. There is also a Lys-plasminogen formed *in vivo* by hydrolysis at amino acids 76-77 of Glu-plasminogen as shown in SEQ ID NO: 6, and the cDNA sequence encoding this amino acid sequence is shown in SEQ ID NO: 5. Delta-plasminogen (δ-plasminogen) is a fragment of the full-length plasminogen with a deletion of Kringle2-Kringle5 structure, and comprises only Kringle1 and the serine protease domain. The amino acid sequence of delta-plasminogen (SEQ ID NO: 8) has been reported in a literature, and the cDNA sequence encoding this amino acid sequence is shown in SEQ ID NO: 7. Mini-plasminogen is composed of Kringle5 and the serine protease domain, and it is reported in a literature that mini-plasminogen comprises residues Val443-Asn791 (with the Glu residue of the Glu-plasminogen sequence without the signal peptide as the starting amino acid), its amino acid sequence is shown in SEQ ID NO: 10, and the cDNA sequence encoding this amino acid sequence is shown in SEQ ID NO: 9. Micro-plasminogen only comprises the serine protease domain, it is reported in a literature that the amino acid sequence of micro-plasminogen comprises residues Ala543-Asn791 (with the Glu residue of the Glu-plasminogen sequence without the signal peptide as the starting amino acid); CN102154253A discloses that its sequence comprises residues Lys531-Asn791 (with the Glu residue of the Glu-plasminogen sequence without the signal peptide as the starting amino acid). For the sequence of micro-plasminogen in the present patent, referring to the patent document CN102154253A, its amino acid sequence is shown in SEQ ID NO: 12, and the cDNA sequence encoding this amino acid sequence is shown in SEQ ID NO: 11.

In this invention, terms "plasmin", "fibrinolysin", and "fibrinolytic enzyme" can be used interchangeably and have the same meaning; terms "plasminogen", "profibrinolysin", and "fibrinolytic zymogen" can be used interchangeably and have the same meaning.

In the application, the meaning of the "deficiency" of plasminogen or plasminogen activity is that the content of plasminogen in the subject is lower than that of a normal person, and it is low enough to affect the normal physiological function of the subject; the meaning of "lack" of plasminogen or plasminogen activity is that the content of plasminogen in the subject is significantly lower than that of normal people, and even the activity or expression is minimal, and normal physiological functions can only be maintained through external sources.

In the circulation process, plasminogen adopts a closed inactive conformation, but when binding to the surface of thrombus or cell, it transforms to active plasmin with an open conformation under the mediation of plasminogen activator (PA). The active plasmin can further hydrolyze the fibrin clot into fibrin degradation products and D-dimers, thereby dissolving the thrombus. The PAp domain of plasminogen comprises important determinants that maintain plasminogen in an inactive closed conformation, while the KR domain can bind to lysine residues present on the receptor and substrate. A variety of enzymes known to act as plasminogen activators include: tissue plasminogen activator (tPA), urokinase plasminogen activator (uPA), kallikrein, coagulation factor XII (Hagerman factor), etc.

The term "plasminogen active fragment" used herein encompasses: 1) an active fragment in the plasminogen protein that can bind to the target sequence in the substrate, which is also called lysine-binding fragment, for example, a fragment comprising Kringle 1, Kringle 2, Kringle 3, Kringle 4, and/or Kringle 5 (the structure of the plasminogen is described in Aisina R B, Mukhametova L I. Structure and function of plasminogen/plasmin system [J]. Russian Journal of Bioorganic Chemistry, 2014, 40(6):590-605); 2) an active fragment in plasminogen protein that exhibits proteolytic function, for example, a fragment having the plasminogen activity (proteolytic function) with a sequence as shown in SEQ ID NO: 14; 3) a fragment in plasminogen protein that has both the activity of binding target sequence in the substrate (lysine binding activity) and the plasminogen activity (proteolytic function). In some embodiments of the application, the plasminogen is a protein comprising the lysine binding fragments of Kringle 1, Kringle 2, Kringle 3, Kringle 4, and/or Kringle 5. Therefore, the plasminogen of the present invention comprises a protein having the active fragment of the plasminogen and still retaining the activity of the plasminogen. In some embodiments, the plasminogen of the application comprises Kringle 1, Kringle 2, Kringle 3, Kringle 4, and/or Kringle 5, or a protein having at least 80%, 90%, 95%, 96%, 97%, 98%, 99% homology with Kringle 1, Kringle 2, Kringle 3, Kringle 4, or Kringle 5 and still having lysine binding activity.

At present, the methods for measuring plasminogen and its activity in blood include: detection of tissue plasminogen activator activity (t-PAA), detection of plasma tissue plasminogen activator antigen (t-PAAg), detection of plasma tissue plasminogen activity (plgA), detection of plasma tissue plasminogen antigen (plgAg), detection of inhibitor activity of plasma tissue plasminogen activator, detection of inhibitor antigen of plasma tissue plasminogen activator, and detection of plasma plasmin-antiplasmin complex (PAP). The most commonly used detection method is the chromogenic substrate method, which includes: adding streptokinase (SK) and chromogenic substrate to the plasma to be tested, wherein the PLG in the plasma to be tested is transformed into PLM under the action of SK, and the latter acts on the chromogenic substrate; subsequently measuring with a spectrophotometer, wherein the increase in absorbance is proportional to the activity of plasminogen. In addition, immunochemical methods, gel electrophoresis, immunoturbidimetry, radioimmuno-diffusion methods, etc. can also be used to determine the plasminogen activity in the blood.

The term "orthologue or ortholog" refers to a homolog between different species, including both protein homolog and DNA homolog, and they are also known as orthologous homolog and vertical homolog; the term particularly refers to a protein or gene evolving from the same ancestral gene in different species. The plasminogen of the present invention includes human natural plasminogen, and also includes orthologues or orthologs of plasminogen derived from different species and having plasminogen activity.

"Conservative substitution variant" refers to a protein or enzyme in which a given amino acid residue is changed without altering the overall conformation and function of the protein or enzyme, including but not limited to, replacing an amino acid in the amino acid sequence of the parent protein with an amino acid having similar property (such as acidity, alkalinity, or hydrophobicity, etc.). Amino acids with similar property are well known. For example, arginine, histidine and lysine are hydrophilic basic amino acids, and are interchangeable. Similarly, isoleucine is a hydrophobic amino acid that can be replaced by leucine, methionine, or valine. Therefore, the similarity of two proteins or amino acid sequences with similar functions may be different, for example, the similarity (identity) is 70%-99% based on the MEGALIGN algorithm. "Conservation substitution variant" also includes a polypeptide or enzyme having amino acid identity of 60% or more, preferably 75% or more, more preferably 85% or more, even more preferably 90% or more as determined by the BLAST or FASTA algorithm, and having the same or substantially similar properties or functions as the natural or parent protein or enzyme.

"Isolated" plasminogen refers to a plasminogen protein that is isolated and/or recovered from its natural environment. In some embodiments, the plasminogen will be purified (1) to a purity of greater than 90%, greater than 95%, or greater than 98% (by weight), as determined by the Lowry method, such as more than 99% (by weight); (2) to a degree sufficiently to obtain at least 15 residues of the N terminal or internal amino acid sequence by using a spinning cup sequenator; or (3) to homogeneity, which is determined by sodium dodecyl sulfate-polyacrylamide gel electrophoresis (SDS-PAGE) under reducing or non-reducing conditions by using Coomassie blue or silver staining. Isolated plasminogen also includes a plasminogen prepared from recombinant cells by bioengineering techniques and separated by at least one purification step.

The terms "polypeptide", "peptide" and "protein" are used interchangeably herein, and refer to polymeric forms of amino acids of any length, which may include genetically encoded and non-genetically encoded amino acids, chemically or biochemically modified or derivative amino acids, and polypeptides having modified peptide backbones. The term includes fusion proteins, including but not limited to, fusion proteins having heterologous amino acid sequences, fusions having heterologous and homologous leader sequences (with or without N-terminal methionine residues); and the like.

The "percent amino acid sequence identity (%)" with respect to the reference polypeptide sequence, is defined as the percentage of amino acid residues in the candidate sequence which are identical to the amino acid residues in the reference polypeptide sequence when gaps are introduced as necessary to achieve maximal percent sequence identity, and no conservative substitutions are considered as part of the sequence identity. The comparison for purpose of determining percent amino acid sequence identity can be achieved in a variety of ways within the technical scope of the art, for example, by using publicly available computer software, such as BLAST, BLAST-2, ALIGN or Megalign (DNASTAR) software. Those skilled in the art can determine appropriate parameters for aligning sequences, including any algorithm needed to achieve the maximum comparison over the full length of the sequences being compared. However, for purpose of the present invention, the percent amino acid sequence identity value is generated by using the sequence comparison computer program ALIGN-2.

In the case of comparing amino acid sequences by using ALIGN-2, the % amino acid sequence identity of a given amino acid sequence A relative to another given amino acid sequence B (or it may be expressed as: a given amino acid sequence A having or comprising a certain % amino acid sequence identity relative to, with or for another given amino acid sequence B) is calculated as follows:

$$\text{fraction } X/Y \times 100$$

wherein X is the number of identically matched amino acid residues scored by the sequence alignment program ALIGN-2 in the alignment of sequences A and B, and wherein Y is the total number of amino acid residues in sequence B. It will be appreciated that, where the length of amino acid sequence A is not equal to the length of amino acid sequence B, the % amino acid sequence identity of sequence A relative to sequence B will not be equal to the % amino acid sequence identity of sequence B relative to sequence A. Unless expressly stated otherwise, all the % amino acid sequence identity values used herein are obtained by using the ALIGN-2 computer program as described in the previous paragraph.

As used herein, the terms "treatment/treating" refer to obtaining a desired pharmacological and/or physiologic effect. The effect may be complete or partial prevention of a disease or its symptoms, and/or partial or complete cure of the disease and/or its symptoms, and includes: (a) prevention of the disease from occurring in a subject, which may have a predisposition to the disease but has not been diagnosed as having the disease; (b) suppression of the disease, i.e., blocking its development; and (c) alleviation of the disease and/or its symptoms, i.e., eliminating the disease and/or its symptoms.

The terms "individual", "subject" and "patient" are used interchangeably herein, and refer to mammals, including but not limited to, murine (rats and mice), non-human primates, humans, dogs, cats, hoofed animals (e.g., horses, cattle, sheep, pigs, goats) and the like.

"Therapeutically effective amount" or "effective amount" refers to an amount of plasminogen sufficient to achieve the prevention and/or treatment of a disease, when administered to a mammal or other subject to treat the disease. The "therapeutically effective amount" will vary depending on the plasminogen to be used, the severity of the disease and/or its symptoms, as well as the age, body weight of the subject to be treated, and the like.

**Preparation of Plasminogen of the Present Invention**

Plasminogen can be isolated and purified from nature for further therapeutic uses, and can also be synthesized by standard chemical peptide synthesis techniques. When chemically synthesized, a polypeptide can be subjected to liquid or solid phase synthesis. Solid phase polypeptide synthesis (SPPS) is a method suitable for chemical synthesis of plasminogen, in which the C-terminal amino acid of a sequence is attached to an insoluble support, followed by the sequential addition of the remaining amino acids in the sequence. Various forms of SPPS, such as Fmoc and Boc, can be used to synthesize plasminogen. Techniques for solid phase synthesis are described in Barany and Solid-Phase Peptide Synthesis; pp. 3-284 in The Peptides: Analysis, Synthesis, Biology. Vol. 2: Special Methods in Peptide Synthesis, Part A., Merrifield et al. J. Am. Chem. Soc., 85: 2149-2156 (1963); Stewart et al. Solid Phase Peptide Synthesis, 2nd ed. Pierce Chem. Co., Rockford, Ill. (1984); and Ganesan A. 2006 Mini Rev. Med Chem. 6:3-10 and Camarero JA et al. 2005 Protein Pept Lett. 12:723-8. Briefly, small insoluble porous beads are treated with a functional unit on which a peptide chain is constructed. After repeated cycles of coupling/deprotection, the attached solid phase free N-terminal amine is coupled to a single N-protected amino acid unit. This unit is then deprotected to expose a new N-terminal amine that can be attached to another amino acid. The peptide remains immobilized on the solid phase before it is cut off.

Standard recombinant methods can be used to produce the plasminogen of the present invention. For example, a nucleic acid encoding plasminogen is inserted into an expression vector, so that it is operably linked to a regulatory sequence in the expression vector. Expression regulatory sequence includes, but is not limited to, promoters (e.g., naturally associated or heterologous promoters), signal sequences, enhancer elements, and transcription termination sequences. Expression regulation can be a eukaryotic promoter system in a vector that is capable of transforming or transfecting eukaryotic host cells (e.g., COS or CHO cells). Once the vector is incorporated into a suitable host, the host is maintained under conditions suitable for high-level expression of the nucleotide sequence and collection and purification of plasminogen.

A suitable expression vector is usually replicated in a host organism as an episome, or as an integral part of the host chromosomal DNA. In general, an expression vector comprises a selective marker (e.g., ampicillin resistance, hygromycin resistance, tetracycline resistance, kanamycin resistance or neomycin resistance) to facilitate detection of those exogenous cells transformed with a desired DNA sequence.

*Escherichia coli* is an example of prokaryotic host cell that can be used to clone the encoding polynucleotide of plasminogen. Other microbial hosts suitable for use include *Bacillus* such as *Bacillus subtilis,* and other species of *enterobacteriaceae* such as *Salmonella, Serratia,* and various species of *Pseudomonas.* In these prokaryotic hosts, expression vectors can also be generated, and they will typically comprise an expression control sequence (e.g., origin of replication) that is compatible with the host cell. In addition, there will be many well-known promoters, such as the lactose promoter system, the tryptophan (trp) promoter system, the beta-lactamase promoter system, or the promoter system from phage λ. Optionally in the case of manipulation of a gene sequence, a promoter will usually control expression, and has a ribosome binding site sequence and the like, so as to initiate and complete transcription and translation.

Other microorganisms, such as yeast can also be used for expression. *Saccharomyces* (e.g., *S. cerevisiae*) and *Pichia pastoris* are examples of suitable yeast host cells, in which a suitable vector has an expression control sequence (e.g., promoter), an origin of replication, a termination sequence, and the like, as required. A typical promoter comprises 3-phosphoglycerate kinase and other glycolytic enzymes. Inducible yeast promoters specifically include those derived from ethanol dehydrogenase, isocytochrome C, and enzymes responsible for maltose and galactose utilization.

In addition to microorganisms, mammalian cells (e.g., mammalian cells cultured *in vitro* in cell culture material) may also be used to express and produce plasminogen (e.g., a polynucleotide encoding plasminogen) of the present invention. See Winnacker, From Genes to Clones, VCH Publishers, N.Y., N.Y. (1987). Suitable mammalian host cells include CHO cell lines, various Cos cell lines, HeLa cells, myeloma cell lines, and transformed B cells or hybridomas. Expression vectors for these cells may comprise an expression control sequence, such as an origin of replication, promoter and enhancer (Queen et al. Immunol. Rev. 89:49 (1986)), as well as necessary information processing sites, such as ribosome binding site, RNA splice site, polyadenylation site, and transcription terminator sequence. Examples of suitable expression control sequences are promoters derived from immunoglobulin gene, SV40, adenovirus, bovine papilloma virus, cytomegalovirus, and the like. See Co et al. J. Immunol. 148:1149 (1992).

Once synthesized (chemically or recombinantly), the plasminogen of the present invention can be purified according to standard procedures in the art, including ammonium sulfate precipitation, affinity column, column chromatography, high performance liquid chromatography (HPLC), gel electrophoresis, and the like. The plasminogen is substantially pure, e.g., with a purity of at least about 80% to 85%, at least about 85% to 90%, at least about 90% to 95%, or 98% to 99% or more, for example, free of contaminants, such as cell debris, macromolecules other than plasminogen, and the like.

**Pharmaceutical Formulations**

A therapeutic formulation can be prepared by mixing plasminogen having a desired purity with an optional pharmaceutical carrier, excipient, or stabilizer (Remington's Pharmaceutical Sciences, 16th edition, Osol, A. ed. (1980)) to form a lyophilized preparation or an aqueous solution. Acceptable carriers, excipients and stabilizers are non-toxic to the recipient at the used dosages and concentrations, and include buffers, such as phosphates, citrates and other organic acids; antioxidants, including ascorbic acid and methionine; preservatives (e.g., octadecyl dimethyl benzyl ammonium chloride; hexanediamine chloride; benzalkonium chloride and benzethonium chloride; phenol, butanol, or benzyl alcohol; alkyl p-hydroxybenzoates, such as methyl or propyl p-hydroxybenzoate; catechol; resorcinol; cyclohexanol; 3-pentanol; and m-cresol); low molecular weight polypeptides (less than about 10 residues); proteins, such as serum albumin, gelatin or immunoglobulins; hydrophilic polymers, such as polyvinylpyrrolidone; amino acids, such as glycine, glutamine, asparagine, histidine, arginine, or lysine; monosaccharides, disaccharides and other carbohydrates, including glucose, mannose, or dextrin; chelating agents, such as EDTA; carbohydrates, such as sucrose, mannitol, fucose, or sorbitol; salt-forming counter ions, such as sodium; metal complexes (e.g., zinc-protein complexes); and/or non-ionic surfactants, e.g., TWEEN™, PLURONICS™, or polyethylene glycol (PEG).

The formulations of the invention may also comprise one or more active compounds required for the particular disorder to be treated, preferably those that are complementary in activity and have no side effects with one another, for instance, medicaments for treating hypertension, arrhythmia, and diabetes, etc.

The plasminogen of the present invention may be encapsulated in microcapsules prepared by techniques such as coacervation technique or interfacial polymerization, for example, it may be incorporated in a colloid drug delivery system (e.g., liposomes, albumin microspheres, microemulsions, nanoparticles and nanocapsules), or incorporated in hydroxymethyl cellulose or gel-microcapsules and poly-(methyl methacrylate) microcapsules in macroemulsion. These techniques are disclosed in Remington's Pharmaceutical Sciences, 16th edition, Osol, A. Ed. (1980).

The plasminogen of the present invention for *in vivo* administration must be sterile. This can be easily achieved by filtration through a filtration membrane for sterilizing, before or after lyophilization and reformulation.

The plasminogen of the present invention can be prepared into a sustained-release preparation. Suitable examples of sustained-release preparations include semi-permeable matrices of solid hydrophobic polymer which have a certain shape and comprise glycoproteins, for example, films or microcapsules. Examples of sustained-release matrices include polyesters, hydrogels (e.g., poly(2-hydroxyethyl-methacrylate)) (Langer et al. J. Biomed. Mater. Res., 15: 167-277 (1981); and Langer, Chem. Tech., 12:98-105 (1982)), or poly(vinyl alcohol), polylactides (US Patent 3,773,919, and EP 58,481), copolymer of L-glutamic acid and y ethyl-L-glutamic acid (Sidman et al., Biopolymers 22:547(1983)), non-degradable ethylene-vinyl acetate (Langer et al. *supra*), or degradable lactic acid-glycolic acid copolymers such as Lupron Depot™ (injectable microspheres composed of lactic acid-glycolic acid copolymer and leuprolide acetate), and poly D-(-)-3-hydroxybutyric acid. Polymers, such as ethylene-vinyl acetate and lactic acid-glycolic acid, are able to persistently release the molecules for 100 days or longer, while some hydrogels release proteins for a shorter period of time. A rational strategy for stabilizing the proteins can be designed according to relevant mechanisms. For example, if the aggregation mechanism is discovered to form intermolecular S-S bonds through thio-disulfide interchange, then the stability may be achieved by modifying sulfhydryl residues, lyophilizing from acidic solutions, controlling moisture content, using appropriate additives, and developing specific polymer matrix compositions.

**Administration and Dosage**

The pharmaceutical composition of the present invention can be administered in different ways, for example by intravenous, intraperitoneal, subcutaneous, intracranial, intrathecal, intraarterial (e.g., via carotid), intramuscular administration.

Preparations for parenteral administration include sterile aqueous or non-aqueous solutions, suspensions and emulsions. Examples of non-aqueous solvents are propylene glycol, polyethylene glycol, vegetable oils such as olive oil, and injectable organic esters such as ethyl oleate. Aqueous carriers include water, and alcoholic/aqueous solutions, emulsions or suspensions, including saline and buffered media. Parenteral vehicles include sodium chloride solution, Ringer's dextrose, dextrose and sodium chloride, or fixed oils. Intravenous vehicles include liquid and nutrient supplements, electrolyte supplements, and the like. Preservatives and other additives may also be present, for example, such as antimicrobial agents, antioxidants, chelating agents and inert gases, etc.

The medical staff will determine the dosage regimen based on various clinical factors. As well known in the medical field, the dosage of any patient depends on a variety of factors, including the patient's size, body surface area, age, the specific compound to be administered, sex, frequency and route of administration, general health, and other medicaments administered simultaneously. The dosage range of the pharmaceutical composition comprising plasminogen of the present invention may be about 0.0001-2000 mg/kg, or about 0.001-500 mg/kg (e.g., 0.02 mg/kg, 0.25 mg/kg, 0.5 mg/kg, 0.75 mg/kg, 10 mg/kg, 50 mg/kg, etc.) body weight of the subject per day. For example, the dosage may be 1 mg/kg body weight, or 50 mg/kg body weight, or in the range of 1-50 mg/kg, or at least 1 mg/kg. Dosages above or below this exemplary range are also contemplated, especially considering the above factors. The intermediate dosages in the above range are also included in the scope of the present invention. A subject may be administered with such dosages daily, every other day, weekly or based on any other schedule determined by empirical analysis. An exemplary dosage schedule includes 1-10 mg/kg for consecutive days. During administration of the medicament of the present invention, the therapeutic effect and safety are required to be assessed real-timely.

**A Product or Kit**

One embodiment of the present disclosure relates to a product or kit comprising the plasminogen of the present invention for treating cardiovascular diseases and related disorders caused by diabetes. The product preferably comprises a container, label or package insert.

Suitable containers include bottles, vials, syringes, and the like. The container can be made of various materials, such as glass or plastic. The container contains a composition that is effective to treat the disease or disorder of the present invention, and has a sterile access (for example, the container may be an intravenous solution bag or vial containing a plug that can be pierced by a hypodermic injection needle). At least one active agent in the composition is plasminogen.

The label on or attached to the container indicates that, the composition is used for treating cardiovascular diseases and related disorders caused by diabetes of the present invention. The product may further comprise a second container containing a pharmaceutically acceptable buffer, such as phosphate buffered saline, Ringer's solution and glucose solution. It may further comprise other substances required from a commercial and user perspective, including other buffers, diluents, filters, needles and syringes. In addition, the product comprises a package insert with instructions for use, including, for example, instructions to a user of the composition to administer the plasminogen composition and other medicaments for treating an accompanying disease to a patient.

EXAMPLE

**Example 1: Plasminogen prolongs the lifespan and median survival of amyotrophic lateral sclerosis model mice**

The human plasminogen used in this example is derived from donor plasma, and is obtained by purification based on the method described in the literatures [1-3] with process optimization. The purity of human plasminogen monomer is >95%. The human plasminogen used in all the following examples is the same.

The transgenic mutant SOD1 has the histopathological characteristics clinically observed in the sporadic and familial amyotrophic lateral sclerosis (ALS). The ALS model mice of the application are B6.Cg-Tg(SOD1-G93A)1Gur/J transgenic mice (abbreviated as SOD1-G93A), purchased from Jackson Laboratory, and animal-related experiments are conducted in an SPF environment. The SOD1-G93A model mice develop hind limb tremor on about day 100, and then the condition quickly deteriorate, with a 50% survival rate of 157.1±9.3 days [4]. At present, it has been widely used in the study of the ALS mechanism and the pre-clinical trial research for new medicament development of ALS.

Nine 16-week old SOD1-G93A male mice are taken, and randomly divided into two groups according to their body weights, 5 in the vehicle control group and 4 in the plasminogen group. The mice in the vehicle control group are injected with the vehicle (PBS, pH7.4) in the tail vein at 0.1 ml/day, and the mice in the plasminogen group are injected with plasminogen in the tail vein at 1 mg/0.1 ml/mouse/day. The survival of the mice is observed and recorded every day.

The results show that, the average life span of the mice in the plasminogen group is 164±8.6 days, the average life span of the mice in the vehicle control group is 153±0 days, and the life span of the mice in the plasminogen group is about 11 days longer than that of the mice in the vehicle control group (Fig. 1A); the median survival time of the mice administered plasminogen is 53±9 days, the median survival time of the mice in the vehicle control group is 40±0 days, and the median survival time of the mice in the plasminogen group is about 13 days longer than that of the mice in the vehicle control group, about 30% longer (Fig. 1B). This result indicates that, plasminogen can prolong the lifespan and median survival of ALS mice.

**Example 2: Plasminogen improves neuromuscular function of ALS model mice**

Nine 16-week old SOD1-G93A male mice are taken, and the randomly divided into two groups according to their body weights, 5 mice in the vehicle control group and 4 mice in the plasminogen group. The mice in the vehicle control group are injected with the vehicle (PBS, pH7.4) in the tail vein at 0.1 ml/day, and the mice in the plasminogen group are injected with plasminogen in the tail vein at 1 mg/0.1 ml/mouse/day for 34 consecutive days, and the start time of administration is set as day 0. The suspension grip strength test is performed on the days 6, 8, 12, 16, 21, 23, 27, 30, and 34 after administration, so as to investigate the effect of plasminogen on the neuromuscular function of ALS model mice, and statistical analysis of neurobehavioral performance of ALS mice is performed as follows.

Suspension Grip Strength Test

The suspension experiment is generally used to evaluate the motor ability (muscle strength) of the mice. A single mouse is placed on the metal lid of the mouse cage, and the lid is gently shaken to make the mouse grasp the lid, then turning over the lid; and the latent time of loosening the mouse's two hind limbs is recorded [5]. Three experiments are performed for each mouse, the longest duration of a single experiment is 90s, and the longest latent time of each mouse is used for the statistical analysis.

The results show that, although the suspension latent time of the mice in the two groups is decreasing during the administration period, the suspension latent time of the mice in the plasminogen group is always longer than that of the mice in the vehicle control group, and on the days 6, 21, and 23 of administration, the statistical difference of the suspension latent time between the plasminogen group and the vehicle group is significant or extremely significant, with the P values of 0.03, 0.02, and 0.008, respectively (Fig. 2). It indicates that, plasminogen can delay the muscle strength loss of ALS mice.

ALS dyskinesia characterization scores, 0 point: no signs of motor dysfunction; 1 point: significant tremor in the hind limbs during tail suspension; 2 points: abnormal gait, toes curl at least 2 times during a 75cm walk, or legs drag along the bottom of the cage; 3 points: the hind limbs have been dragged for at least 1 day, stiff and weak (rigid paralysis), and the legs cannot be used for forward movement; 4 points: the mouse is placed in the supine position, and it cannot turn over to the prone position within 30 seconds ( determined as dying state) [5].

The results show that, the time point of the 2 points neurobehavior score of the mice in the plasminogen group is significantly later than that of the mice in the vehicle group, and the statistical difference is significant (* means P<0.05) (Fig. 3).

The above results indicate that, plasminogen can significantly delay the muscle strength loss of ALS disease and delay the progression of ALS disease.

**Example 3: Plasminogen slows down the weight loss of ALS model mice**

20 wild-type mice with similar week old ages, 29 male SOD1-G93A mice, and 31 female SOD1-G93A mice are taken. The wild-type mice are used as a blank control group (no administration treatment is done). SOD1-G93A mice are observed and recorded when their legs tremble occurs after the onset of the disease in the 14th week, the time of onset of each mouse is recorded, and the administration is started 14 days after the onset of disease; all mice are randomly divided into a vehicle control group and a plasminogen group according to the onset of disease, wherein 32 mice in the vehicle control group are injected with the vehicle (10 mM sodium citrate buffer, pH7.4) in the tail vein at 0.1 ml/mouse/day; and 28 mice in the plasminogen group are injected with plasminogen in the tail vein at 1 mg/0.1 ml/mouse/day, for 35 consecutive days in an SPF environment. The start time of administration is set as day 0, and the body weight is measured every 3 days during the administration period, so as to investigate the effect of plasminogen on the weight loss of ALS model mice.

ALS is usually accompanied by significant weight loss, which is one of the main characteristics of ALS [5]. The results of each weight measurement are standardized with the weight on day 0, that is: each weight measurement value/weight on day 0 *100.

The results show that during administration, the weight of the mice in the blank control group does not fluctuate much with a gradual upward trend; the weight of the mice in the vehicle control group gradually decreases; the weight of the mice in the plasminogen group fluctuates greatly in the first 25 days, but all is close to or slightly larger than the body weight of the mice in the blank control group; the body weight gradually decreases after 25 days, but it is always greater than that of the mice in the vehicle control group, and compared with the vehicle control group, the P value is less than or close to 0.001 (Fig. 4). It shows that, plasminogen can significantly reduce the rate of weight loss in ALS model mice, and delay the deterioration of ALS.

**Example 4: Plasminogen reduces the vacuolar area of the anterior horn of spinal cord of ALS model mice**

Five wild-type male mice with similar week old ages and nine male SOD1-G93A mice are taken. The wild-type mice are used as a blank control group. SOD1-G93A mice are observed and recorded when their legs tremble occurs after the onset of the disease in the 14th week, and the time of onset of each mouse is recorded, the administration is started 14 days after the onset of disease; all mice are randomly divided into a vehicle control group and an administration group according to the onset of disease, wherein 5 mice in the vehicle control group are injected with the vehicle (10 mM sodium citrate buffer, pH7.4) in the tail vein at 0.1 ml/mouse/day; and 4 mice in the administration group are injected with plasminogen in the tail vein at 1 mg/0.1 ml/mouse/day continuously in an SPF environment, the material is taken when the mouse is dying, and the longest administration is 61 days. The spinal cord is taken and fixed in formalin fixative. The fixed tissue is dehydrated by gradient alcohol, made transparent with xylene, and embedded in paraffin. The tissue slices have a thickness of 3 $\mu$m, they are deparaffinized, rehydrated, washed once with water, and stained with hematoxylin dye for 10 minutes, then rinsed in running water for 5 minutes, after differentiating with 1% hydrochloric acid and ethanol for 10 seconds, rinsing in running water for 10 minutes, then staining in 0.2% eosin dye solution for 10 seconds; finally they are dehydrated by gradient alcohol, made transparent and sealed. The slices are observed under a 400x optical microscope.

The degeneration and death of motor neurons in the anterior horn of spinal cord is one of the main pathological features of ALS [6]. The results show that, the anterior horn of spinal cord of the blank control mice (Fig. 5A) exhibits a certain level of vacuolar area, and the vacuolar area of the anterior horn of spinal cord of the mice in the vehicle group (Fig. 5B) is significantly larger than that of the mice in the blank control (P<0.001). The vacuolar area of the anterior horn of spinal cord of the mice in the administration group (Fig. 5C) is significantly lower than that of the mice in the vehicle group, and the statistical difference is extremely significant (Fig. 5D). It indicates that, plasminogen can reduce the vacuolar area of the anterior horn of spinal cord, and reduce the death of motor neurons in the anterior horn of spinal cord in ALS model mice.

**Example 5: Plasminogen promotes the expression of acetylcholine transferase in the anterior horn of spinal cord of ALS model mice**

Five wild-type male mice with similar week old ages and nine male SOD1-G93A mice are taken. The wild-type mice are used as a blank control group. SOD1-G93A mice are observed and recorded when their legs tremble occurs after the onset of the disease in the 14th week, and the time of onset of each mouse is recorded, the administration is started 14 days after the onset of disease; all mice are randomly divided into a vehicle control group and an administration group according to the onset of disease, wherein 5 mice in the vehicle control group are injected with the vehicle (10 mM sodium citrate buffer, pH7.4) in the tail vein at 0.1 ml/mouse/day; and 4 mice in the administration group are injected with plasminogen in the tail vein at 1 mg/0.1 ml/mouse/day continuously in an SPF environment, the material is taken when the mouse is dying, and the longest administration is 61 days. The spinal cord is taken and fixed in formalin fixative. The fixed tissue is dehydrated by gradient alcohol, made transparent with xylene, and embedded in paraffin. The tissue slices have a thickness of 3 $\mu$m, they are deparaffinized, rehydrated, and washed once with water. The tissue is circled with PAP pen, then incubating with 3% hydrogen peroxide for 15 minutes, and washing twice with 0.01 M PBS, 5 minutes for each time. After blocking with 5% normal goat serum (Vector laboratories, Inc., USA) for 30 minutes, the goat serum is discarded, and rabbit-derived anti-acetylcholine transferase antibody (ab178850, Abcam) is added dropwise to incubate overnight at 4°C, then washing twice with 0.01 M PBS, 5 minutes for each time. The secondary antibody of goat anti-rabbit IgG (HRP) antibody (Abcam) is incubated for 1 hour at room temperature, then washing twice with 0.01 M PBS, 5 minutes for each time. The color is developed according to DAB kit (Vector laboratories, Inc., USA), washing 3 times with water, then counterstaining with hematoxylin dye for 10 minutes, and rinsing in running water for 5 minutes. Finally the slices are dehydrated by gradient alcohol, made transparent with xylene, and sealed with neutral gum. The slices are observed under a 400x optical microscope.

Acetylcholine transferase (chAT) is a marker enzyme of cholinergic neurons, and is synthesized in nerve cells. Studies have shown that, the chAT level in motor neurons of animal spinal cord of ALS animal model and in the patients is reduced [7,8].

The results show that, a certain amount of chAT is expressed in the anterior horn of spinal cord in the blank control mice (Fig. 6A), the expression level of chAT in the anterior horn of spinal cord of the mice in the vehicle group (Fig. 6B) is significantly lower than that of the mice in the blank control group, the expression level of chAT in the anterior horn of spinal cord of the mice in the administration group (Fig. 6C) is significantly higher than that of the mice in the vehicle group, and the statistical difference is significant (P<0.05) (Fig. 6D). It indicates that TP01HN106 can promote the synthesis and expression of chAT in the anterior horn of spinal cord of SOD1-G93A mice, and promote the recovery of cholinergic neuron function.

**Example 6: Plasminogen promotes the expression of synaptophysin in the anterior horn of spinal cord of ALS model mice**

Five wild-type male mice with similar week old ages and nine male SOD1-G93A mice are taken. The wild-type mice are used as a blank control group. SOD1-G93A mice are observed and recorded when their legs tremble occurs after the onset of the disease in the 14th week, and the time of onset of each mouse is recorded, the administration is started 14 days after the onset of disease; all mice are randomly divided into a vehicle control group and an administration group according to the onset of disease, wherein 5 mice in the vehicle control group are injected with the vehicle (10 mM sodium citrate buffer, pH7.4) in the tail vein at 0.1 ml/ mouse/day; and 4 mice in the administration group are injected with plasminogen in the tail vein at 1 mg/0.1 ml/mouse/day continuously in an SPF environment, the material is taken when the mouse is dying, and the longest administration is 61 days. The spinal cord is taken and fixed in formalin fixative. The fixed tissue is dehydrated by gradient alcohol, made transparent with xylene, and embedded in paraffin. The tissue slices have a thickness of 3 μm, they are deparaffinized, rehydrated, and washed once with water. The tissue is circled with PAP pen, then incubating with 3% hydrogen peroxide for 15 minutes, and washing twice with 0.01 M PBS, 5 minutes for each time. After blocking with 5% normal goat serum (Vector laboratories, Inc., USA) for 30 minutes, the goat serum is discarded, and rabbit anti-synapsin antibody (17785-1-AP, Proteintech) is added dropwise to incubate overnight at 4°C, then washing twice with 0.01 M PBS, 5 minutes for each time. The secondary antibody of goat anti-rabbit IgG (HRP) antibody (Abcam) is incubated for 1 hour at room temperature, then washing twice with 0.01 M PBS, 5 minutes for each time. The color is developed according to DAB kit (Vector laboratories, Inc., USA), washing 3 times with water, then counterstaining with hematoxylin dye for 10 minutes, and rinsing in running water for 5 minutes. Finally the slices are dehydrated by gradient alcohol, made transparent with xylene, and sealed with neutral gum. The slices are observed under a 400x optical microscope.

Synaptophysin is a phosphorylated protein on the presynaptic membrane, as a sign of axon growth and synapse formation, and it is closely related to synaptic flexibility. ALS model mice show obvious synaptic degeneration and loss of motor neuron cell bodies before clinical symptoms appear [9].

The results show that, a certain level of synaptophysin is expressed in the anterior horn of spinal cord of the mice in the blank control group (Fig. 7A), the expression level of synaptophysin in the vehicle group (Fig. 7B) is significantly lower than that of the mice in the blank control group; the expression level of synaptophysin in the anterior horn of spinal cord of the mice in the administration group (Fig. 7C) is significantly higher than that of the mice in the vehicle group, and the statistical difference is significant (P<0.05) (Fig. 7D). It indicates that, plasminogen can promote the expression of synaptophysin in the anterior horn of spinal cord of the model mice, and promote the repair of synaptic damage.

**Example 7: Plasminogen promotes the repair of inflammation in the anterior horn of spinal cord in ALS model mice**

Five wild-type male mice with similar week old ages and nine male SOD1-G93A mice are taken. The wild-type mice are used as a blank control group. SOD1-G93A mice are observed and recorded when their legs tremble occurs after the onset of the disease in the 14th week, and the time of onset of each mouse is recorded, the administration is started 14 days after the onset of disease; all mice are randomly divided into a vehicle control group and an administration group according to the onset of disease, wherein 5 mice in the vehicle control group are injected with the vehicle (10 mM sodium citrate buffer, pH7.4) in the tail vein at 0.1 ml/mouse/day; and 4 mice in the administration group are injected with plasminogen in the tail vein at 1 mg/0.1 ml/mouse/day continuously in an SPF environment, the material is taken when the mouse is dying, and the longest administration is 61 days. The spinal cord is taken and fixed in formalin fixative. The fixed tissue is dehydrated by gradient alcohol, made transparent with xylene, and embedded in paraffin. The slices have a thickness of 3 $\mu$m, they are deparaffinized, rehydrated, and washed once with water. The tissue is circled with PAP pen, then incubating with 3% hydrogen peroxide for 15 minutes, and washing twice with 0.01 M PBS, 5 minutes for each time. After blocking with 5% normal goat serum (Vector laboratories, Inc., USA) for 30 minutes, the goat serum is discarded, and rabbit anti-Iba-1 (ab178847, Abcam) is added dropwise to incubate overnight at 4°C, then washing twice with 0.01 M PBS, 5 minutes for each time. The secondary antibody of goat anti-rabbit IgG (HRP) antibody (Abcam) is incubated for 1 hour at room temperature, then washing twice with 0.01 M PBS, 5 minutes for each time. The color is developed according to DAB kit (Vector laboratories, Inc., USA), washing 3 times with water, then counterstaining with hematoxylin dye for 10 minutes, and rinsing in running water for 5 minutes. Finally the slices are dehydrated by gradient, made transparent with xylene, and sealed with neutral gum. The slices are observed under a 400x optical microscope.

Ionized calcium binding adaptor molecule-1 (Iba-1) is a surface marker of microglia in the central nervous system. As the immune cells in the central nervous system, microglia can quickly sense neurological disorders and be activated when they are pathological or injured. The activated microglia have significant changes in number and morphology, and migrate to the injured site to perform a variety of functions, for example, phagocytosis of dead cells, and increased production of pro-inflammatory cytokines, and the like [10].

The results show that, a certain level of Iba-1 is expressed in the anterior horn of spinal cord of the blank control mice (Fig. 8A), the expression level of Iba-1 in the anterior horn of spinal cord of the mice in the administration group (Fig. 8C) is significantly higher than that of the mice in the vehicle group (Fig. 8B) and the blank control group, and the statistical difference is significant (P<0.05 or 0.01). It indicates that plasminogen can promote the repair of inflammation in the anterior horn of spinal cord in the model mice.

**Example 8: Plasminogen ameliorates muscle atrophy in ALS model mice**

Five wild-type male mice with similar week old ages and nine male SOD1-G93A mice are taken. The wild-type mice are used as a blank control group. SOD1-G93A mice are observed and recorded when their legs tremble occurs after the onset of the disease in the 14th week, and the time of onset of each mouse is recorded, the administration is started 14 days after the onset of disease; all mice are randomly divided into a vehicle control group and an administration group according to the onset of disease, wherein 5 mice in the vehicle control group are injected with the vehicle (10 mM sodium citrate buffer, pH7.4) in the tail vein at 0.1 ml/mouse/day; and 4 mice in the administration group are injected with plasminogen in the tail vein at 1 mg/0.1 ml/mouse/day continuously in an SPF environment, the material is taken when the mouse is dying, and the longest administration is 61 days. The gastrocnemius muscle is taken and fixed in formalin fixative. The fixed tissue is dehydrated by gradient alcohol, made transparent with xylene, and embedded in paraffin. The tissue slices have a thickness of 3 $\mu$m, they are deparaffinized, rehydrated, washed once with water, and stained with hematoxylin dye for 10 minutes, then rinsed in running water for 5 minutes, after differentiating with 1% hydrochloric acid and ethanol for 10 seconds, rinsing in running water for 10 minutes, then staining in 0.2% eosin dye solution for 10 seconds; finally they are dehydrated by gradient alcohol, made transparent and sealed. The slices are observed under a 200x optical microscope.

The results show that, the gastrocnemius fibers in the blank control group (Fig. 9A) are complete in structure and relatively uniform in shape and size, while the gastrocnemius fibers in the vehicle group (Fig. 9B) show severe atrophy, accompanying with local inflammatory cell infiltration (red arrow) and roundness change of muscle fibers. The muscle fiber atrophy in the administration group (Fig. 9C) is less severe than that in the vehicle group, but there was also inflammatory cell infiltration. It indicates that plasminogen can ameliorate muscle atrophy in the model mice.

**Example 9: Plasminogen ameliorates muscle atrophy in ALS model mice**

Five wild-type male mice with similar week old ages and nine male SOD1-G93A mice are taken. The wild-type mice are used as a blank control group. SOD1-G93A mice are observed and recorded when their legs tremble occurs after the onset of the disease in the 14th week, and the time of onset of each mouse is recorded, the administration is started 14 days after the onset of disease; all mice are randomly divided into a vehicle control group and an administration group according to the onset of disease, wherein 5 mice in the vehicle control group are injected with the vehicle (10 mM sodium citrate buffer, pH7.4) in the tail vein at 0.1 ml/mouse/day; and 4 mice in the administration group are injected with plasminogen in the tail vein at 1 mg/0.1 ml/mouse/day continuously in an SPF environment, the material is taken when the mouse is dying, and the longest administration is 61 days. The gluteus muscle is taken and fixed in formalin fixative. The fixed tissue is dehydrated by gradient alcohol, made transparent with xylene, and embedded in paraffin. The tissue slices have a thickness of 3 μm, they are deparaffinized, rehydrated, washed once with water, and stained with hematoxylin dye for 10 minutes, then rinsed in running water for 5 minutes, after differentiating with 1% hydrochloric acid and ethanol for 10 seconds, rinsing in running water for 10 minutes, then staining in 0.2% eosin dye solution for 10 seconds; finally they are dehydrated by gradient alcohol, made transparent and sealed. The slices are observed under a 200x optical microscope.

The results show that, the muscle fibers in the blank control group (Fig. 10A) mice are relatively complete in structure and relatively uniform in shape and size. The muscle fibers of the gluteal muscle of the mice in the vehicle group (Fig. 10B) show roundness change, different sizes, severe atrophy, accompanying with infiltration of inflammatory cells. The structure of the gluteal muscle fibers of the mice in the administration group (Fig. 10C) recovers to a certain extent compared with that in the vehicle group. It is suggested that plasminogen can ameliorate muscle atrophy in the model mice.

**Example 10: Plasminogen promotes the expression of SMN protein in the anterior horn of spinal cord of ALS model mice**

Five wild-type male mice with similar week old ages and nine male SOD1-G93A mice are taken. The wild-type mice are used as a blank control group. SOD1-G93A mice are observed and recorded when their legs tremble occurs after the onset of the disease in the 14th week, and the time of onset of each mouse is recorded, the administration is started 14 days after the onset of disease; all mice are randomly divided into a vehicle control group and an administration group according to the onset of disease, wherein 5 mice in the vehicle control group are injected with the vehicle (10 mM sodium citrate buffer, pH7.) in the tail vein at 0.1 ml/ mouse/day; and 4 mice in the administration group are injected with plasminogen in the tail vein at 1 mg/0.1 ml/mouse/day continuously in an SPF environment, the material is taken when the mouse is dying, and the longest administration is 61 days. The spinal cord is taken and fixed in formalin fixative. The fixed tissue is dehydrated by gradient alcohol, made transparent with xylene, and embedded in paraffin. The tissue slices have a thickness of 3 μm, they are deparaffinized, rehydrated, and washed once with water. The tissue is circled with PAP pen, then incubating with 3% hydrogen peroxide for 15 minutes, and washing twice with 0.01 M PBS, 5 minutes for each time. After blocking with 5% normal goat serum (Vector laboratories, Inc., USA) for 30 minutes, the goat serum is discarded, and rabbit-derived anti-SMN antibody (Abcam) is added dropwise to incubate overnight at 4°C, then washing twice with 0.01 M PBS, 5 minutes for each time. The secondary antibody of goat anti-rabbit IgG (HRP) antibody (Abcam) is incubated for 1 hour at room temperature, then washing twice with 0.01 M PBS, 5 minutes for each time. The color is developed according to DAB kit (Vector laboratories, Inc., USA), washing 3 times with water, then counterstaining with hematoxylin dye for 10 minutes, and rinsing in running water for 5 minutes. Finally the slices are dehydrated by gradient alcohol, made transparent with xylene, and sealed with neutral gum. The slices are observed under a 400x optical microscope.

Studies on survival motor neuron (SMN) protein show that, the level of survival motor neuron (SMN) protein is decreased in SOD1-ALS model, and the increase of SMN protein can ameliorate the disease phenotype [11].

The results show that, the expression level of SMN protein in the anterior horn of spinal cord of the mice in the administration group (Fig. 11B) is significantly higher than that in the vehicle group (Fig. 11A). It indicates that plasminogen can promote the expression of SMN protein in the anterior horn of spinal cord of model mice.

REFERENCES

[1] KENNETH C. ROBBINS, LOUIS SUMMARIA, DAVID ELWYN et al. Further Studies on the Purification and Characterization of Human Plasminogen and Plasmin. Journal of Biological Chemistry, 1965, 240 (1) :541-550.

[2] Summaria L, Spitz F, Arzadon L et al. Isolation and characterization of the affinity chromatography forms of human Glu- and Lys-plasminogens and plasmins. J Biol Chem. 1976 Jun 25;251(12):3693-9.

[3] HAGAN JJ, ABLONDI FB, DE RENZO EC. Purification and biochemical properties of human plasminogen. J Biol Chem. 1960 Apr;235: 1005-10.

[4] Dobrowolny, G. Muscle expression of a local Igf-1 isoform protects motor neurons in an ALS mouse model [J]. The Journal of Cell Biology, 2005, 168(2):193-199.

[5] Weydt P, Hong S Y, Kliot M, et al. Assessing disease onset and progression in the SOD1 mouse model of ALS.[J]. Neuroreport, 2003, 14(7):1051-4.

[6] L. M. Murray, K. Talbot, T. H. Gillingwater. Review: Neuromuscular synaptic vulnerability in motor neurone disease: amyotrophic lateral sclerosis and spinal muscular atrophy[J]. neuropathology & applied neurobiology, 2010, 36(2):133-156.

[7] Michael L. Berger, Mario Veitl, Susanne Malessa et al. Cholinergic markers in ALS spinal cord[J]. Journal of the Neurological Sciences, 1992, 108(1):114.

[8] Jordan K, Murphy J, Singh A, et al. Astrocyte-Mediated Neuromodulatory Regulation in Preclinical ALS: A Metadata Analysis[J]. Frontiers in Cellular Neuroscience, 2018, 12.

[9] L. M. Murray, K. Talbot, T. H. Gillingwater. Review: Neuromuscular synaptic vulnerability in motor neurone disease: amyotrophic lateral sclerosis and spinal muscular atrophy[J]. neuropathology & applied neurobiology, 2010, 36(2):133-156.

[10]Min, Kyoung-Jin, Yang, Myung-Soon, et al. Protein kinase A mediates microglial activation induced by plasminogen and gangliosides [J]. Experimental & Molecular Medicine, 2004, 36(5):461-467.

[11] L. M. Murray, K. Talbot, T. H. Gillingwater. Review: Neuromuscular synaptic vulnerability in motor neurone disease: amyotrophic lateral sclerosis and spinal muscular atrophy [J]. neuropathology & applied neurobiology, 2010, 36(2):133-156.

## Claims

1.  A pharmaceutical composition comprising a therapeutically effective amount of a plasminogen pathway activator for use in treating amyotrophic lateral sclerosis (ALS), in a subject suffering from amyotrophic lateral sclerosis (ALS), wherein the plasminogen pathway activator is plasminogen.

2.  The pharmaceutical composition for use according to claim 1, wherein the plasminogen pathway activator has one or more activities in the subject suffering from amyotrophic lateral sclerosis (ALS) and the one or more activities are selected from the group consisting of: prolonging life span and median survival, delaying muscle atrophy and muscle strength loss, slowing down the rate of weight loss, reducing cell damage, degeneration and necrosis in the anterior horn of spinal cord, promoting the synthesis of chAT in the anterior horn of spinal cord, promoting the recovery of cholinergic neuron function, promoting the expression of synaptophysin in the anterior horn of spinal cord, promoting the expression of SMN protein in the anterior horn of spinal cord, promoting the repair of inflammation in the anterior horn of spinal cord, and promoting the repair of synaptic damage.

3.  The pharmaceutical composition for use according to claim 1, wherein the plasminogen pathway activator ameliorates the symptoms of muscle atrophy, muscle strength loss, spasm, and/or fasciculation in the subject.

4.  The pharmaceutical composition for use according to claim 1, wherein the plasminogen pathway activator reduces weight loss and/or prolongs survival in the subject.

5.  The pharmaceutical composition for use according to claim 1, wherein the plasminogen pathway activator improves muscle tone in the subject.

6.  The pharmaceutical composition for use according to claim 1, wherein the plasminogen pathway activator promotes the recovery of muscle function in the subject.

7.  The pharmaceutical composition for use according to claim 1, wherein the plasminogen pathway activator promotes the repair of neuron damage in the anterior horn of spinal cord in the subject.

8.  The pharmaceutical composition for use according to any one of claims 1-7, wherein the plasminogen pathway activator is administered in combination with one or more other medicaments and/or therapies.

9.  The pharmaceutical composition for use according to any one of claims 1-8, wherein the plasminogen pathway activator is administered by intravenous, subcutaneous, intramuscular, intrathecal, nasal inhalation, aerosol inhalation, nasal drop or eye drop administration.

10. The pharmaceutical composition for use according to claims 1 to 9, wherein the plasminogen has at least 80%, 85%, 90%, 95%, 96%, 97%, 98% or 99% sequence identity with SEQ ID NOs: 2, 6, 8, 10 or 12 and has plasminogen activity.

11. The pharmaceutical composition for use according to claim 10, wherein the plasminogen is a protein that is an active fragment of plasminogen and has plasminogen activity and/or lysine binding activity.

12. The pharmaceutical composition for use according to claim 10, wherein the plasminogen is selected from the group consisting of: Glu-plasminogen, Lys-plasminogen, mini-plasminogen, micro-plasminogen, delta-plasminogen.

13. The pharmaceutical composition for use according to claim 10, wherein the plasminogen is natural or synthetic human plasminogen, or fragment thereof retaining plasminogen activity and/or lysine binding activity.

## Patentansprüche

1.  Pharmazeutische Zusammensetzung, umfassend eine therapeutisch wirksame Menge eines Plasminogenweg-Aktivators zur Verwendung bei der Behandlung von amyotropher Lateralsklerose (ALS) bei einem an amyotropher Lateralsklerose (ALS) leidenden Patienten, wobei der Plasminogenweg-Aktivator Plasminogen ist.

2.  Pharmazeutische Zusammensetzung zur Verwendung gemäß Anspruch 1, wobei der Plasminogenweg-Aktivator eine oder mehrere Aktivitäten in dem an amyotropher Lateralsklerose (ALS) leidenden Patienten aufweist, und die eine oder mehreren Aktivitäten ausgewählt sind aus der Gruppe bestehend aus: Verlängerung der Lebensspanne und des Überlebens-Medians, Verzögerung der Muskelatrophie und des Muskelkraftverlustes, Verlangsamung der Rate des Gewichtsverlustes, Verringerung der Zellschädigung, der Degeneration und der Nekrose im Vorderhorn des Rückenmarks, Förderung der Synthese von chAT im Vorderhorn des Rückenmarks, Förderung der Wiederherstellung der Funktion der cholinergen Neuronen, Förderung der Expression von Synaptophysin im Vorderhorn des Rückenmarks, Förderung der Expression des SMN-Proteins im Vorderhorn des Rückenmarks, Förderung der Behebung von Entzündungen im Vorderhorn des Rückenmarks und Förderung der Reparatur von synaptischen Schäden.

3. Pharmazeutische Zusammensetzung zur Verwendung gemäß Anspruch 1, wobei der Plasminogenweg-Aktivator die Symptome der Muskelatrophie, des Muskelkraftverlusts, der Spasmen und/oder der Faszikulation bei dem Patienten verbessert.

4. Pharmazeutische Zusammensetzung zur Verwendung gemäß Anspruch 1, wobei der Plasminogenweg-Aktivator den Gewichtsverlust verringert und/oder das Überleben des Patienten verlängert.

5. Pharmazeutische Zusammensetzung zur Verwendung gemäß Anspruch 1, wobei der Plasminogenweg-Aktivator den Muskeltonus bei dem Patienten verbessert.

6. Pharmazeutische Zusammensetzung zur Verwendung gemäß Anspruch 1, wobei der Plasminogenweg-Aktivator die Wiederherstellung der Muskelfunktion bei dem Patienten fördert.

7. Pharmazeutische Zusammensetzung zur Verwendung gemäß Anspruch 1, wobei der Plasminogenweg-Aktivator die Behebung von Neuronenschäden im Vorderhorn des Rückenmarks bei dem Patienten fördert.

8. Pharmazeutische Zusammensetzung zur Verwendung gemäß einem der Ansprüche 1 bis 7, wobei der Plasminogenweg-Aktivator in Kombination mit einem oder mehreren anderen Medikamenten und/oder Therapien verabreicht wird.

9. Pharmazeutische Zusammensetzung zur Verwendung gemäß einem der Ansprüche 1 bis 8, wobei der Plasminogenweg-Aktivator intravenös, subkutan, intramuskulär, intrathekal, durch nasale Inhalation, durch Aerosol-Inhalation, als Nasentropfen oder als Augentropfen verabreicht wird.

10. Pharmazeutische Zusammensetzung zur Verwendung gemäß einem der Ansprüche 1 bis 9, wobei das Plasminogen mindestens 80 %, 85 %, 90 %, 95 %, 96 %, 97 %, 98 % oder 99 % Sequenzübereinstimmung mit den SEQ ID NOs: 2, 6, 8, 10 oder 12 aufweist und Plasminogenaktivität aufweist.

11. Pharmazeutische Zusammensetzung zur Verwendung gemäß Anspruch 10, wobei das Plasminogen ein Protein ist, das ein aktives Fragment von Plasminogen ist und Plasminogenaktivität und/oder Lysinbindungsaktivität aufweist.

12. Pharmazeutische Zusammensetzung zur Verwendung gemäß Anspruch 10, wobei das Plasminogen ausgewählt ist aus der Gruppe bestehend aus: Glu-Plasminogen, Lys-Plasminogen, Mini-Plasminogen, Mikro-Plasminogen, Delta-Plasminogen.

13. Pharmazeutische Zusammensetzung zur Verwendung gemäß Anspruch 10, wobei das Plasminogen natürliches oder synthetisches menschliches Plasminogen oder ein Fragment davon ist, das Plasminogenaktivität und/oder Lysinbindungsaktivität beibehält.

**Revendications**

1. Composition pharmaceutique comprenant une quantité thérapeutiquement efficace d'un activateur de la voie du plasminogène destiné à être utilisé dans le traitement de la sclérose latérale amyotrophique (SLA), chez un sujet souffrant de sclérose latérale amyotrophique (SLA), dans laquelle l'activateur de la voie du plasminogène est le plasminogène.

2. Composition pharmaceutique destinée à être utilisée selon la revendication 1, dans laquelle l'activateur de la voie du plasminogène a une ou plusieurs activités chez le sujet souffrant de sclérose latérale amyotrophique (SLA) et l'une ou plusieurs activités sont choisies dans le groupe consistant en : prolonger la durée de vie et la survie médiane, retarder l'atrophie musculaire et la perte de force musculaire, ralentir le taux de perte de poids, réduire les dommages cellulaires, la dégénérescence et la nécrose dans la corne antérieure de la moelle épinière, favoriser la synthèse de chAT dans la corne antérieure de la moelle épinière, favoriser la récupération de la fonction des neurones cholinergiques, favoriser l'expression de la synaptophysine dans la corne antérieure de la moelle épinière, favoriser l'expression de la protéine SMN dans la corne antérieure de la moelle épinière, favoriser la réparation de l'inflammation dans la corne antérieure de la moelle épinière et favoriser la réparation des dommages synaptiques.

3. Composition pharmaceutique destinée à être utilisée selon la revendication 1, dans laquelle l'activateur de la voie du plasminogène améliore les symptômes d'atrophie musculaire, de perte de force musculaire, de spasme et/ou de fasciculation chez le sujet.

4. Composition pharmaceutique destinée à être utilisée selon la revendication 1, dans laquelle l'activateur de la voie du plasminogène réduit la perte de poids et/ou prolonge la survie chez le sujet.

5. Composition pharmaceutique destinée à être utilisée selon la revendication 1, dans laquelle l'activateur de la voie du plasminogène améliore le tonus musculaire chez le sujet.

6. Composition pharmaceutique destinée à être utilisée selon la revendication 1, dans laquelle l'activateur de la voie du plasminogène favorise la récupération de la fonction musculaire chez le sujet.

7. Composition pharmaceutique destinée à être utilisée selon la revendication 1, dans laquelle l'activateur de la voie du plasminogène favorise la réparation des lésions neuronales dans la corne antérieure de la moelle épinière chez le sujet.

8. Composition pharmaceutique destinée à être utilisée selon l'une quelconque des revendications 1 à 7, dans laquelle l'activateur de la voie du plasminogène est administré en combinaison avec un ou plusieurs autres médicaments et/ou thérapies.

9. Composition pharmaceutique destinée à être utilisée selon l'une quelconque des revendications 1 à 8, dans laquelle l'activateur de la voie du plasminogène est administré par voie intraveineuse, sous-cutanée, intramusculaire, intrathécale, nasale, aérosol, goutte nasale ou collyre.

10. Composition pharmaceutique destinée à être utilisée selon les revendications 1 à 9, dans laquelle le plasminogène a au moins 80 %, 85 %, 90 %, 95 %, 96 %, 97 %, 98 % ou 99 % d'identité de séquence avec les SEQ ID NO: 2, 6, 8, 10 ou 12 et a une activité plasminogène.

11. Composition pharmaceutique destinée à être utilisée selon la revendication 10, dans laquelle le plasminogène est une protéine qui est un fragment actif de plasminogène et qui a une activité de plasminogène et/ou une activité de liaison à la lysine.

12. Composition pharmaceutique destinée à être utilisée selon la revendication 10, dans laquelle le plasminogène est choisi dans le groupe constitué par : Glu-plasminogène, Lys-plasminogène, mini-plasminogène, micro-plasminogène, delta-plasminogène.

13. Composition pharmaceutique destinée à être utilisée selon la revendication 10, dans laquelle le plasminogène est du plasminogène humain naturel ou synthétique, ou un fragment de celui-ci conservant une activité de plasminogène et/ou une activité de liaison à la lysine.

Fig. 1A

Fig. 1B

Fig. 2

Fig. 3

Fig. 4

Fig. 5

Fig. 6

Fig. 7

Fig. 8

Fig. 9

Fig. 10

Fig. 11

**REFERENCES CITED IN THE DESCRIPTION**

**Patent documents cited in the description**

- CN 102154253 A
- US 3773919 A
- EP 58481 A


**Non-patent literature cited in the description**

- **KIERNAN MC ; VUCIS S ; CHEAH BC et al.** Amyotrophic lateral sclerosis. *Lancet*, 2011, vol. 377, 942-955
- **NY, A. ; LEONARDSSON, G. ; HAGGLUND, A.C ; HAGGLOF, P. ; PLOPLIS, V.A ; CARMELIET, P. ; NY, T.** Ovulation inplasminogen-deficient mice.. *Endocrinology*, 1999, vol. 140, 5030-5035
- **SILVERSTEIN RL ; LEUNG LL ; HARPEL PC ; NACHMAN RL.** Complex formation of platelet thrombospondin with plasminogen. Modulation of activation by tissue activator. *J. Clin. Invest.*, November 1984, vol. 74 (5), 1625-33
- **GRAVANIS I ; TSIRKA SE.** Tissue-type plasminogen activator as a therapeutic target in stroke. *Expert Opinion on Therapeutic Targets.*, February 2008, vol. 12 (2), 159-70
- **GEIGER M ; HUBER K ; WOJTA J ; STINGL L ; ESPANA F ; GRIFFIN JH ; BINDER BR.** Complex formation between urokinase and plasma protein C inhibitor in vitro and in vivo. *Blood*, August 1989, vol. 74 (2), 722-8
- **AISINA R B ; MUKHAMETOVA L I.** Structure and function of plasminogen/plasmin system [J].. *Russian Journal of Bioorganic Chemistry*, 2014, vol. 40 (6), 590-605
- **BARANY.** *Solid-Phase Peptide Synthesis*, 3-284
- The Peptides: Analysis, Synthesis, Biology.. *Special Methods in Peptide Synthesis*, vol. 2
- **MERRIFIELD et al.** *J. Am. Chem. Soc.*, 1963, vol. 85, 2149-2156
- **STEWART et al.** Solid Phase Peptide Synthesis. Pierce Chem. Co., 1984
- **GANESAN A.** *Mini Rev. Med Chem.*, 2006, vol. 6, 3-10
- **CAMARERO JA et al.** *Protein Pept Lett.*, 2005, vol. 12, 723-8
- **WINNACKER.** From Genes to Clones. VCH Publishers, 1987
- **QUEEN et al.** *Immunol. Rev.*, 1986, vol. 89, 49
- **CO et al.** *J. Immunol.*, 1992, vol. 148, 1149
- *Remington's Pharmaceutical Sciences*, 1980
- Remington's Pharmaceutical Sciences. 1980
- **LANGER et al.** *J. Biomed. Mater. Res*, 1981, vol. 15, 167-277
- **LANGER.** *Chem. Tech.*, 1982, vol. 12, 98-105
- **SIDMAN et al.** *Biopolymers*, 1983, vol. 22, 547
- **KENNETH C. ROBBINS ; LOUIS SUMMARIA ; DAVID ELWYN et al.** Further Studies on the Purification and Characterization of Human Plasminogen and Plasmin.. *Journal of Biological Chemistry*, 1965, vol. 240 (1), 541-550
- **SUMMARIA L ; SPITZ F ; ARZADON L et al.** Isolation and characterization of the affinity chromatography forms of human Glu- and Lys-plasminogens and plasmins.. *J Biol Chem.*, 25 June 1976, vol. 251 (12), 3693-9
- **HAGAN JJ ; ABLONDI FB ; DE RENZO EC.** Purification and biochemical properties of human plasminogen.. *J Biol Chem.*, April 1960, vol. 235, 1005-10
- **DOBROWOLNY, G.** Muscle expression of a local Igf-1 isoform protects motor neurons in an ALS mouse model [J].. *The Journal of Cell Biology*, 2005, vol. 168 (2), 193-199
- **WEYDT P ; HONG S Y ; KLIOT M et al.** Assessing disease onset and progression in the SOD1 mouse model of ALS.[J].. *Neuroreport*, 2003, vol. 14 (7), 1051-4
- **L. M. MURRAY ; K. TALBOT ; T. H. GILLINGWATER.** Review: Neuromuscular synaptic vulnerability in motor neurone disease: amyotrophic lateral sclerosis and spinal muscular atrophy[J].. *neuropathology & applied neurobiology*, 2010, vol. 36 (2), 133-156
- **MICHAEL L. BERGER ; MARIO VEITL ; SUSANNE MALESSA et al.** Cholinergic markers in ALS spinal cord[J].. *Journal of the Neurological Sciences*, 1992, vol. 108 (1), 114
- **JORDAN K ; MURPHY J ; SINGH A et al.** Astrocyte-Mediated Neuromodulatory Regulation in Preclinical ALS: A Metadata Analysis[J].. *Frontiers in Cellular Neuroscience*, 2018, 12

- **MIN, KYOUNG-JIN** ; **YANG, MYUNG-SOON et al.** Protein kinase A mediates microglial activation induced by plasminogen and gangliosides [J].. *Experimental & Molecular Medicine*, 2004, vol. 36 (5), 461-467

- **L. M. MURRAY** ; **K. TALBOT** ; **T. H. GILLINGWATER**. Review: Neuromuscular synaptic vulnerability in motor neurone disease: amyotrophic lateral sclerosis and spinal muscular atrophy [J].. *neuropathology & applied neurobiology*, 2010, vol. 36 (2), 133-156